(19) Europäisches Patentamt
European Patent Office
Office européen des brevets

(11) Numéro de publication: **0 366 560 A1**

(12) **DEMANDE DE BREVET EUROPEEN**

(21) Numéro de dépôt: **89402969.3**

(51) Int. Cl.5: **C07H 17/08 , A61K 31/70**

(22) Date de dépôt: **27.10.89**

Revendication pour l'Etat contractant suivant: ES + GR.

(30) Priorité: **27.10.88 FR 8814004**

(43) Date de publication de la demande: **02.05.90 Bulletin 90/18**

(84) Etats contractants désignés: **AT BE CH DE ES FR GB GR IT LI LU NL SE**

(71) Demandeur: **ADIR ET COMPAGNIE**
**22, rue Garnier**
**F-92201 Neuilly sur Seine(FR)**

(72) Inventeur: **Lukacs, Gabor**
**22 Boulevard Kellerman**
**F-75013 Paris(FR)**

Inventeur: **Ruggeri-Duchatelle, Catherine**
**1 place Jacques Froment**
**F-75018 Paris(FR)**
Inventeur: **Dessinges, Aimée**
**5 Q Boulevard Rocheplatte**
**F-45000 Orléans(FR)**
Inventeur: **Qlesker, Alain**
**Résidence "Les Fonds Fanettes" Batiment 7**
**F-91190 Gif-sur-Yvette(FR)**
Inventeur: **Laborde, Maria**
**6 rue Boyer Barret**
**F-75014 Paris(FR)**
Inventeur: **Ming, Li**
**Chez Madame Prévost 73 rue du Général Leclerc**
**F-91190 Gif-sur-Yvette(FR)**

(54) **Nouveaux dérivés de la tylosine, leur procédé de préparation et les compositions pharmaceutiques qui les contiennent.**

(57) Composés de formule générale :

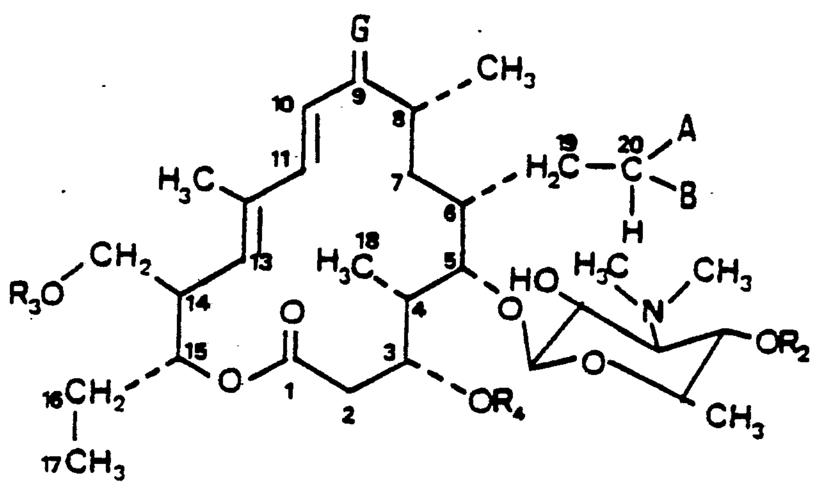

(I)

où A, B, G, R2, R3, R4 et ∿ sont définis dans la description.
Médicaments

EP 0 366 560 A1

# NOUVEAUX DERIVES DE LA TYLOSINE, LEUR PROCEDE DE PREPARATION ET LES COMPOSITIONS PHARMACEUTIQUES QUI LES CONTIENNENT

La présente invention concerne de nouveaux antibiotiques de la famille des macrolides, leur procédé de préparation et les compositions pharmaceutiques qui les contiennent.

Les besoins de la thérapeutique exigent le développement constant de nouveaux antibiotiques à la fois en raison de la possibilité d'apparition de nouvelles souches résistantes, mais également dans le but de créer de nouvelles molécules possédant une activité améliorée aussi bien au niveau de leur seuil d'efficacité que de l'étendue de leur spectre d'action.

De nombreuses modifications du noyau de la tylosine ont déjà été réalisées afin de produire de nouveaux antibiotiques intéressants. Parmi les plus récentes, citons les brevets US 4 528 369, 4 581 346, 4 629 786 et les demandes de brevets européens 0 103 465, 0 104 028, 0 154 495 et 0 203 621. Toutefois, aucune de ces modifications n'a permis d'obtenir un dérivé de la tylosine utilisé en thérapeutique humaine.

Plus particulièrement, la présente invention a pour objet les produits dérivés de la tylosine de formule générale :

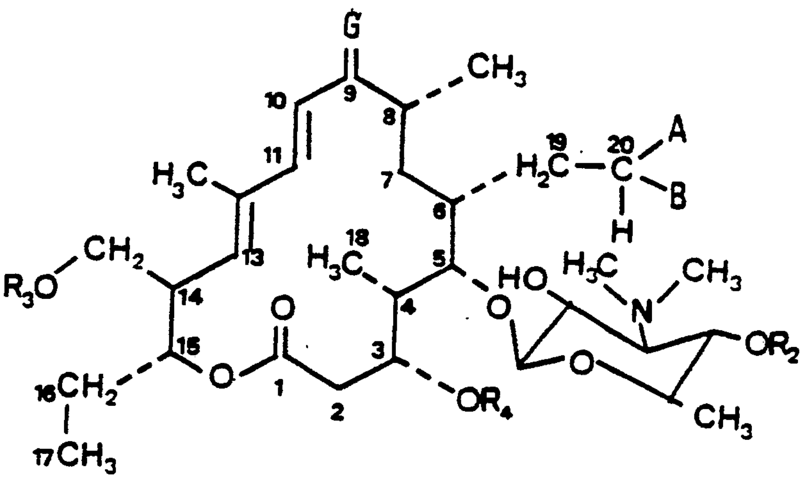

(I)

dans laquelle :

ou bien :

A représente un groupement

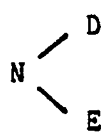

dans lequel D et E identiques ou différents représentent indépendamment l'un de l'autre un atome d'hydrogène, un radical alcoyle inférieur linéaire ou ramifié, éventuellement substitué par un groupement phényle et dans ce cas B représente un groupement cyano,

ou bien :

A et B représentent ensemble un groupement = $N^+(O^-)$ - D dans lequel D a la même signification que précédemment,

G représente un atome d'oxygène ou un groupement de formule N ∿ O - $R_1$ dans lequel $R_1$ représente un atome d'hydrogène, un groupement alcoyle inférieur linéaire ou ramifié, un groupement alkényle inférieur linéaire ou ramifié, ceux-ci étant optionnellement substitué par un groupement phényle lui-même éventuellement substitué par un groupement nitro,

le signe ∿ existant dans la définition de G signifiant que la fonction oxime ou éther d'oxime peut se trouver sous la forme syn ou anti ou sous forme du mélange syn et anti,

$R_2$ représente . soit un atome d'hydrogène,

. soit un radical de formule :

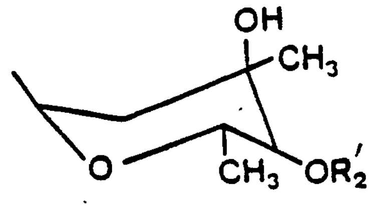

dans lequel $R'_2$ représente un atome d'hydrogène, un radical alcoyle inférieur linéaire ou ramifié ou un radical acyle inférieur, linéaire ou ramifié.
$R_3$ représente un atome d'hydrogène ou un groupement :

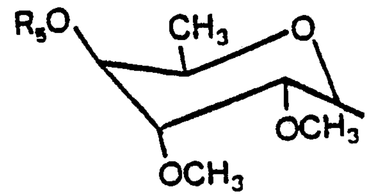

$R_4$ et $R_5$ représentent indépendamment l'un de l'autre :
. soit un atome d'hydrogène,
. soit un radical alcoyle inférieur linéaire ou ramifié.

Par radicaux alcoyle inférieur et alkényle inférieur, on entend des groupements comprenant entre 1 et 6 atomes de carbone.

L'invention s'étend également aux sels des composés de formule (I). Parmi les acides que l'on peut ajouter aux composés de formule (I) pour former un sel d'addition, on peut citer, à titre d'exemple, les acides chlorhydrique, bromhydrique, iodhydrique, sulfurique, acétique, propionique, trifluoroacétique, maléique, malique, tartrique, méthanesulfonique, éthanesulfonique, benzènesulfonique, p-toluènesulfonique, phosphorique, fumarique, citrique, camphorique...

La présente invention s'étend également à un procédé de préparation des dérivés de formule (I) caractérisé en ce que l'on utilise comme matière première un dérivé de formule (II) :

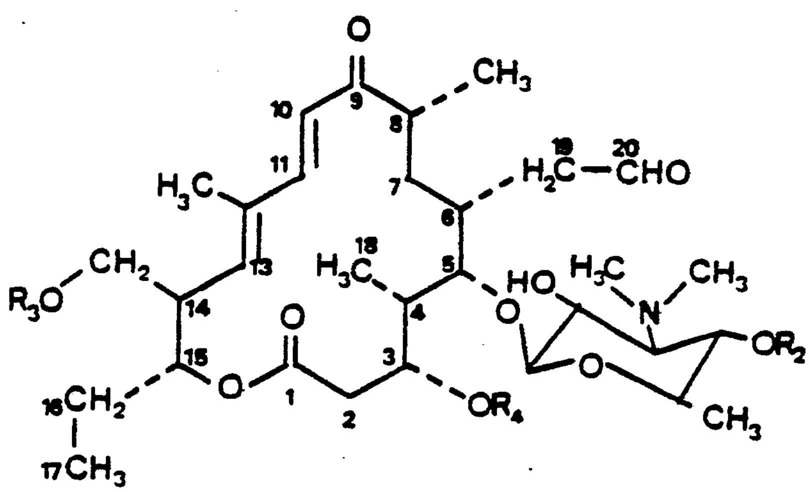

(II)

dans laquelle $R_2$, $R_3$, $R_4$ ont la même définition que dans la formule (I),
qui, dans le cas où dans le produit de formule (I) que l'on souhaite obtenir $R_2$ représente un atome d'hydrogène, est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

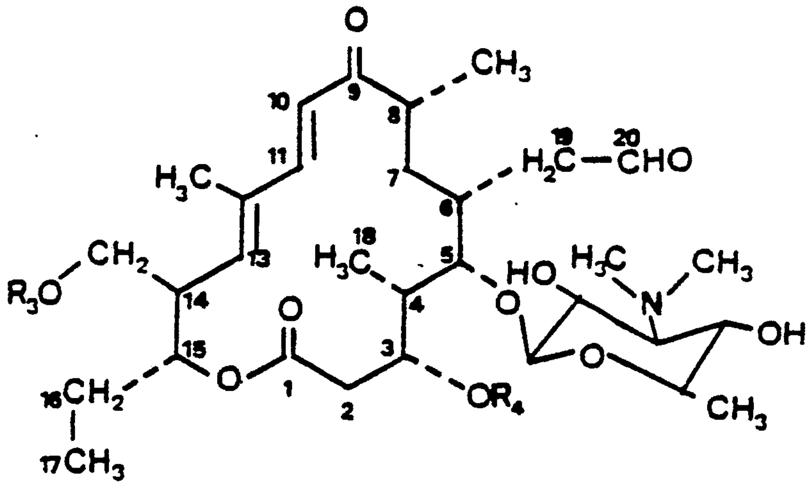

(II/2a)

dans laquelle $R_3$ et $R_4$ ont la même signification que dans la formule (I), qui, dans le cas où dans le produit de formule (I) que l'on souhaite obtenir $R_2$ et $R_3$ simultanément représentent un atome d'hydrogène, est ensuite soumis à réaction avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100 °C, préférentiellement comprise entre 50 et 90 °C, préférentiellement comprise entre 70 et 80 °C, pour conduire, après lavage par un solvant organique approprié, alcalinisation et extraction par un solvant organique approprié à un dérivé de formule (II/2b) :

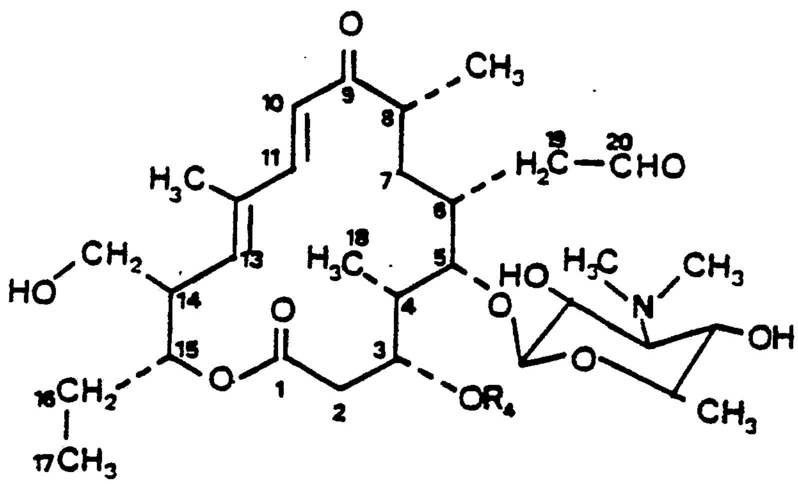

(II/2b)

dans laquelle :
$R_4$ a la même signification que dans la formule (I), que l'on purifie éventuellement par chromatographie sur colonne de silice,
le dérivé alors choisi de formule générale (II), ou (II/2a), ou (II/2b) selon le composé de formule (I) que l'on souhaite obtenir étant alors traité :
- soit, dans le cas où B représente un groupement cyano, par un cyano phosphonate de dialkyle tel que le cyano phosphonate de diéthyle,
en présence d'une amine de formule (III) HNDE où D et E ont la même signification que dans la formule (I) dans un solvant organique, préférentiellement choisi parmi tétrahydrofuranne, éther diéthylique, éther diisopropylique, benzène, dioxanne, acétone, acétate d'éthyle,
à une température comprise inclusivement entre la température ambiante et la température du reflux du solvant choisi pour conduire à un dérivé de formule (I/A), cas particulier des dérivés de formule (I) pour lesquels G représente un atome d'oxygène, A un groupement NDE et B un groupement cyano,

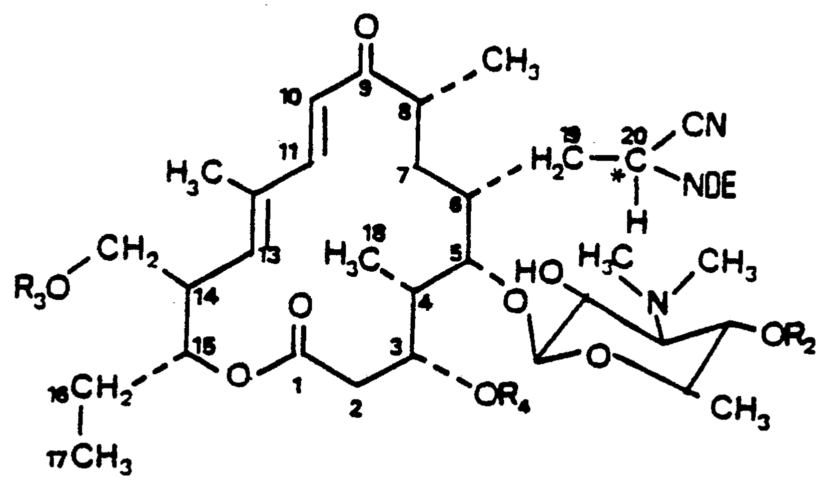

(I/A)

dans laquelle :

D, E et $R_4$ ont la même définition que dans la formule (I), et $R_2$ et $R_3$ la même signification que dans le dérivé choisi (II), (II/2a) ou (II/2b) dont on sépare, si on le désire, les isomères (R) et (S) par une technique classique telle que la chromatographie sur colonne de silice ou la cristallisation fractionnée,

- soit dans le cas où A et B simultanément représentent un groupement = $N^+$ ($O^-$) - D, par un dérivé de formule HO - NHD dans lequel D a la même définition que dans la formule (I), ou bien préférentiellement avec un sel d'acide fort d'un tel produit (chlorhydrate, bromhydrate...) dans un solvant organique préférentiellement choisi parmi alcools aliphatiques inférieurs, acétate d'éthyle, acétonitrile en présence d'une base comme par exemple la pyridine, la triéthylamine ou un sel de métal alcalin tel que l'acétate de sodium ou le carbonate acide de sodium, ou le carbonate acide de potassium, ou le carbonate de sodium, ou le carbonate de potassium, ou le carbonate de calcium pour conduire à un dérivé de formule (I/B) :

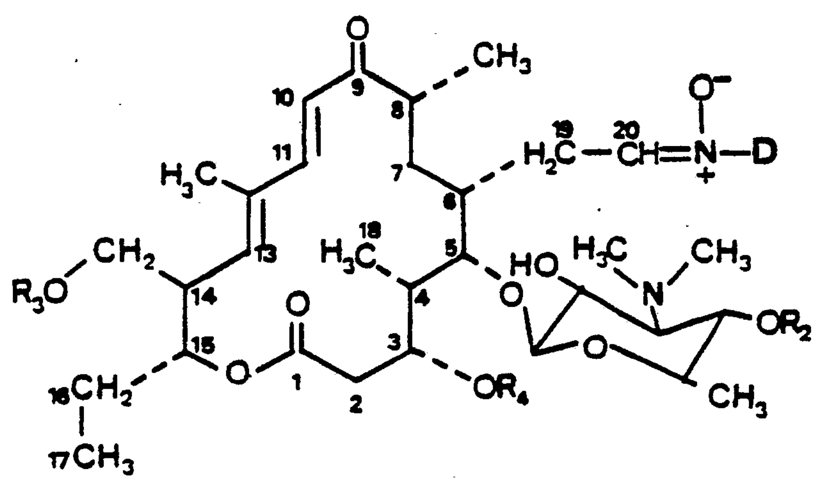

(I/B)

cas particulier des dérivés de formule (I) dans lequel G représente un atome d'oxygène, et A et B représentent simultanément un groupement = $N^+$ -($O^-$) - D,

dans laquelle $R_4$ et D ont la même signification que dans la formule (I) et $R_2$ et $R_3$ la même signification que dans la formule (II), (II/2a) ou (II/2b) du dérivé choisi,

dérivé de formule (I/A) ou (I/B) que l'on traite si on le désire en présence d'une base, comme par exemple la pyridine, la triéthylamine ou un sel de métal alcalin tel que l'acétate de sodium, ou le carbonate acide de sodium, ou le carbonate acide de potassium, ou le carbonate de sodium, ou le carbonate de potassium, ou le carbonate de calcium avec un dérivé de formule (V) :

**$H_2N$ - O - $R_1$ (V)**

dans lequel $R_1$ a la même signification que dans la formule (I) ou bien préférentiellement avec un sel d'acide fort (chlorhydrate, bromhydrate...) d'un tel produit pour obtenir après purification éventuelle par chromatographie sur colonne de silice un dérivé de formule (I/D) :

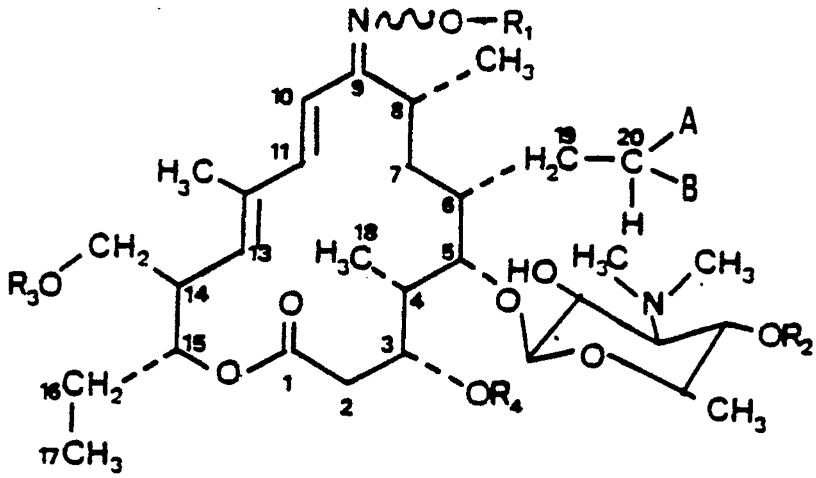

(I/D)

cas particulier des dérivés de formule (I) dans lesquels G représente un groupement N ∿ O - $R_1$ et A, B et $R_4$ ont la même signification que dans le dérivé (I/A) ou (I/B), et $R_2$ et $R_3$ la même signification que dans la formule (II), (II/2a) ou (II/2b) du dérivé choisi,

- que l'on purifie éventuellement par chromatographie sur colonne de silice à l'aide d'un mélange solvant approprié comme par exemple un mélange chlorure de méthylène/méthanol,

et que l'on peut, si on le désire :

- soit salifier par un acide pharmaceutiquement acceptable,
- soit séparer en ses isomères puis, si nécessaire, salifier par un acide pharmaceutiquement acceptable.

Les composés de formule (I) possèdent des propriétés pharmacologiques intéressantes.

En particulier, ces composés sont actifs sur les cocci gram + et cocci gram -, les bacilles gram + (clostridies), certains bacilles gram -, hémophilus (ex : hémophilus influenzae), nesseiria gonorrhoeae, brucella, bordetella, les anaérobies, les mycoplasmes, les rickettsies et les myagawanelles (chlamidia), les spirochètes, les protozoaires et certains dermofongi.

Plus particulièrement, les composés de formule (I) possèdent une très bonne activité antibiotique sur les pneumocoques, les staphylocoques et les streptocoques. Ce spectre d'activité rend les composés de formule (I) particulièrement intéressants dans le traitement d'un grand nombre d'affections ; parmi celles-ci, il est possible de citer, à titre d'exemple, les pneumococcies telles que les bronchites, la brucellose, la diphtérie, la gonococcie, les pneumonies, les streptococcies telles que les angines aiguës, les otites, la scarlatine, les sinusites, les staphylococcies telles que septicémies à staphylocoques, anthrax, érésipèles, pyodermites, staphylococcies aiguës, broncho pneumonies et suppurations pulmonaires.

De plus, les composés de la présente invention, de par leur structure, sont susceptibles de se révéler intéressants en raison de leur absence de toxicité hépatique ou gastrointestinale, ce qui les distingue avantageusement d'autres familles de composés antibiotiques.

La présente invention a également pour objet les compositions pharmaceutiques contenant les produits de formule (I) où un de leurs sels d'addition à un acide pharmaceutiquement acceptable, seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration parentérale, nasale, rectale, perlinguale, oculaire ou respiratoire, et notamment les préparations injectables, les aérosols, les gouttes oculaires ou nasales, les comprimés simples ou dragéifiés, les comprimés sublinguaux, les sachets, les paquets, les gélules, les glossettes, les tablettes, les suppositoires, les crèmes, pommades, gels dermiques, etc...

Les compositions pharmaceutiques selon l'invention peuvent également se présenter sous forme d'une poudre lyophilisée qui est destinée à être dissoute au moment de l'emploi dans un solvant approprié notamment l'eau stérile apyrogène.

La posologie utile varie selon l'âge et le poids du patient, la voie d'administration, la nature de l'indication thérapeutique et des traitements éventuellement associés et s'échelonne entre 1 centigramme et 4 grammes par prise ou par application.

Les exemples suivants illustrent l'invention et ne la limitent en aucune façon.

Les spectres de résonance magnétique nucléaire du $^{13}C$ et du $^{1}H$ ont été enregistrés en utilisant le

TMS comme référence interne.

La matière première utilisée dans la synthèse des composés de formule (I) est la tylosine, connue dans la littérature.

**EXEMPLE 1 : (R,S) DIBENZYLAMINO - 20 CYANO - 20 DESOXO - 20 TYLOSINE**

Dissoudre 9,16 g (10 mmole) de tylosine et 2 ml (12 mmole) de cyano phosphonate de diéthyle dans 200 ml de tétrahydrofuranne anhydre. Ajouter 4,4 ml (22 mmole) de N,N dibenzylamine et agiter à température ambiante pendant 8 heures. Evaporer le milieu réactionnel au bain-marie sous vide et purifier le résidu obtenu par flash chromatographie sur colonne de silice 60 en utilisant comme éluant le mélange de chlorure de méthylène, méthanol, ammoniaque 30/1/0,05.

Rendement : 62 %

Caractéristiques spectrales : R.M.N. $^1H$ ($\delta$ = ppm)

$\delta$ = 7,20 à 7,50 ppm, massif : aromatiques

**EXEMPLE 2 : (R,S) BENZYLOXYIMINO (E + Z) - 9 DIBENZYLAMINO - 20 CYANO - 20 DIDESOXO - 9,20 TYLOSINE**

**STADE A : (R,S) DIBENZYLAMINO - 20 CYANO - 20 DESOXO - 20 TYLOSINE**

Voir exemple 1.

**STADE B : (R, S) BENZYLOXYIMINO (E + Z) - 9 DIBENZYLAMINO - 20 CYANO - 20 DIDESOXO - 9,20 TYLOSINE**

Dissoudre 1,12 g (1 mmole) de (R,S) dibenzylamino (E + Z) - 20 cyano - 20 désoxo - 20 tylosine précédemment obtenue dans 13 ml de pyridine anhydre. Ajouter 480 mg (3 mmole) de chlorhydrate de benzyloxyamine et agiter le mélange réactionnel à température ambiante pendant 4 jours. Ajouter de la glace et extraire au chlorure de méthylène. Sécher la phase organique sur sulfate de sodium, filtrer, évaporer à sec et purifier le résidu obtenu par flash chromatographie sur colonne de silice 60 en utilisant comme éluant le mélange chlorure de méthylène, méthanol, ammoniaque : 40/1/0,05.

Rendement : 45 %

Caractéristiques spectrales : R.M.N. $^1H$ ($\delta$ = ppm)

$\delta$ = 7,3 à 7,6 ppm : massif : aromatiques

**EXEMPLE 3 : (R,S) METHOXYIMINO (E + Z) - 9 DIBENZYLAMINO - 20 CYANO - 20 DIDESOXO - 9,20 TYLOSINE**

En procédant comme dans l'exemple 2, mais en remplaçant au stade B le chlorhydrate de benzyloxyamine par le chlorhydrate de méthoxyamine, on obtient le produit attendu.

Solvant d'élution de la chromatographie sur colonne de silice, chlorure de méthylène, méthanol, ammoniaque : 30/1/0,05.

Rendement : 45 %

Caractéristiques spectrales : R.M.N. $^1H$ ($\delta$ = ppm)

$\delta$ = 3,30 ppm : singulet : 3H, $CH_3$, N - O - $CH_3$

**EXEMPLE 4 : (R,S) HYDROXYIMINO (E + Z) - 9 DIBENZYLAMINO - 20 CYANO - 20 DIDESOXO - 9,20 TYLOSINE**

En procédant comme dans l'exemple 3, mais en remplaçant le chlorhydrate de méthoxyamine par le chlorhydrate d'hydroxylamine, on obtient le produit attendu.

**EXEMPLES 5 A 7 :**

De la même façon que dans l'exemple 3, en remplaçant le chlorhydrate méthoxyamine par :
**EXEMPLE 5 :** le chlorhydrate d'O allylhydroxylamine,
**EXEMPLE 6 :** le chlorhydrate d'O éthylhydroxylamine,
**EXEMPLE 7 :** le chlorhydrate de paranitrobenzyloxyamine, on obtient :

**EXEMPLE 5 : (R,S) ALLYLOXYIMINO (E + Z) - 9 DIBENZYLAMINO - 20 CYANO - 20 DIDESOXO - 9,20 TYLOSINE**

**EXEMPLE 6 : (R,S) ETHYLOXYIMINO (E + Z) - 9 DIBENZYLAMINO - 20 CYANO - 20 DIDESOXO - 9,20 TYLOSINE**

**EXEMPLE 7 : (R,S) PARANITROBENZYLOXYIMINO (E + Z) - 9 DIBENZYLAMINO - 20 CYANO - 20 DIDESOXO - 9,20 TYLOSINE**

**EXEMPLE 8 : N BENZYL NITRONE EN 20 DE LA DESOXO - 20 TYLOSINE**

Dissoudre 1,92 g (2,09 mmole) de tylosine base dans 50 ml d'éthanol. Ajouter 250 mg de bicarbonate de sodium et 335 mg (2,09 mmole) de chlorhydrate de N benzyl hydroxylamine. Agiter le mélange réactionnel à température ambiante pendant 1H30 et évaporer au bain-marie sous vide. Purifier le résidu obtenu par flash chromatographie sur colonne de silice 60 en utilisant comme éluant le mélange chlorure de méthylène, méthanol, ammoniaque 20/1/0,05.
Rendement : 75 %
Caractéristiques spectrales : R.M.N.$^1$H ($\delta$ = ppm)
$\delta$ 4,8 : ppm singulet : 2H, $CH_2$ - $C_6H_5$

**EXEMPLE 9 : N BENZYL NITRONE EN 20 DE LA BENZYLOXYIMINO - 9 (E + Z) DIDESOXO - 9,20 TYLOSINE**

**STADE A : N BENZYL NITRONE EN 20 DE LA DESOXO - 20 TYLOSINE**

Obtenue dans l'exemple 8.

**STADE B : N BENZYL NITRONE EN 20 DE LA BENZYLOXYIMINO - 9 (E + Z) DIDESOXO - 9,20 TYLOSINE**

Dissoudre 500 mg (0,49 mmole) de N benzyl nitrone en 20 de la désoxo - 20 tylosine obtenue au stade précédent dans 10 ml de pyridine anhydre. Ajouter 235 mg (1,47 mmole) de chlorhydrate de benzyloxyamine. Agiter le mélange réactionnel pendant 2 jours à température ambiante. Ajouter ensuite de la glace et extraire au chlorure de méthylène. Sécher la phase organique sur sulfate de sodium, filtrer et évaporer à sec. Le résidu brut est purifié par chromatographie sur colonne de silice 60 en utilisant comme éluant le mélange chlorure de méthylène/méthanol/ammoniaque/20/1/0,05.
Rendement : 55%
Caractéristiques spectrales : R.M.N.$^1$H ($\delta$ = ppm)
$\delta$ = 5,10, 2 x 2H, $2CH_2$ ($CH_2C_6H_5$)
$\delta$ = 7,40, 2 x 5H, aromatiques

**EXEMPLES 10 à 11 :**

En procédant comme dans l'exemple 9 et en remplaçant au stade B le chlorhydrate de benzyloxyamine par :

**EXEMPLE 10 :** le chlorhydrate de méthoxyamine

**EXEMPLE 11 :** le chlorhydrate de paranitrobenzyloxyamine on obtient :

**EXEMPLE 10 : N BENZYL NITRONE EN 20 DE LA METHOXYIMINO - 9 (E + Z) DIDESOXO - 9,20 TYLOSINE**

**EXEMPLE 11 : N BENZYL NITRONE EN 20 DE LA PARANITROBENZYLOXYIMINO - 9 (E + Z) DIDESOXO - 9,20 TYLOSINE**

**EXEMPLE 12 : (R) BENZYLAMINO - 20 CYANO - 20 DESOXO - 20 TYLOSINE ET (S) BENZYLAMINO - 20 CYANO - 20 DESOXO - 20 TYLOSINE**

Dissoudre 500 mg de tylosine base (0,55 mmole) et 0,11 ml (0,66 mmole) de cyano phosphonate de diéthyle dans 10 ml de tétrahydrofuranne anhydre. Ajouter 0,13 ml de benzylamine et agiter le mélange réactionnel à température ambiante pendant 6 heures. Evaporer le tétrahydrofuranne au bain-marie sous vide. Le résidu brut est purifié par chromatographie sur colonne de silice 60 en utilisant comme éluant le mélange chlorure de méthylène/méthanol/ammoniaque/40/1/0,05, on obtient chacun des deux isomères (R) et (S) avec un rendement de 20 % pour chacun d'eux, puis un mélange des deux isomères.

Rendement global : 50 %

Caractéristiques spectrales : R.M.N.$^1$H ($\delta$ = ppm)

$\delta$ = 7,30 ppm : massif : noyaux aromatiques.

**EXEMPLE 13 : BENZYLOXYIMINO (E + Z) - 9 (R) BENZYLAMINO - 20 CYANO - 20 DIDESOXO - 9,20 TYLOSINE**

**STADE A : (R) BENZYLAMINO - 20 CYANO - 20 DESOXO - 20 TYLOSINE**

Voir exemple 12.

**STADE B : BENZYLOXYIMINO (E + Z) - 9 (R) BENZYLAMINO - 20 CYANO -20 DIDESOXO - 9,20 TYLOSINE**

Dissoudre 1,4 mg (1,36 mmole) de (R) benzylamino - 20 cyano - 20 désoxo - 20 tylosine dans 13 ml de pyridine et ajouter 650 mg (4,1 mmole) de chlorhydrate de benzyloxyamine. Agiter le mélange réactionnel 3 jours à température ambiante, ajouter de la glace et extraire au chlorure de méthylène. Sécher sur sulfate de sodium. Filtrer et évaporer à sec. Le résidu obtenu est purifié par chromatographie sur colonne de silice 60 en utilisant comme éluant le mélange chlorure de méthylène/méthanol/ammoniaque/40/1/0,05).

Rendement : 53 %

Caractéristiques spectrales : R.M.N $^1$H ($\delta$ = ppm)

$\delta$ = 7,30 ppm : massif : noyaux aromatiques.

**EXEMPLE 14 : BENZYLOXYIMINO (E + Z) - 9 (S) BENZYLAMINO - 20 CYANO - 20 DIDESOXO - 9,20 TYLOSINE**

En procédant comme dans l'exemple 13, mais en utilisant à l'issue du stade A la (S) benzylamino - 20 cyano - 20 désoxo - 20 tylosine et non la (R) benzylamino - 20 cyano - 20 désoxo - 20 tylosine, on obtient le produit attendu.

Rendement : 40 %

Caractéristiques spectrales : R.M.N.$^1$H ($\delta$ = ppm)

$\delta$ = 7,30 ppm : massif : noyaux aromatiques.

**EXEMPLE 15 : METHYLOXYIMINO (E + Z) - 9 (R) BENZYLAMINO - 20 CYANO - 20 DIDESOXO - 9,20 TYLOSINE**

**STADE A : (R) BENZYLAMINO - 20 CYANO - 20 DESOXO - 20 TYLOSINE**

Obtenue dans l'exemple 12.

**STADE B : METHOXYIMINO (E + Z) - 9 (R) BENZYLAMINO - 20 CYANO - 20 DIDESOXO - 9,20 TYLOSINE**

Dissoudre 1 g (0,97 mmole) de (R) benzylamino - 20 cyano - 20 désoxo - 20 tylosine dans 10 ml de pyridine. Ajouter 250 mg (3 mmole) de chlorhydrate de méthoxyamine. Agiter le mélange réactionnel 8 jours à température ambiante. Ajouter de la glace, extraire au chlorure de méthylène, sécher sur sulfate de sodium, filtrer et évaporer à sec, le résidu est purifié par flash chromatographie sur colonne de silice 60 en utilisant comme éluant le mélange chlorure de méthylène/méthanol/ammoniaque/40/1/0,05.
Rendement : 25%
Caractéristiques spectrales : R.M.N.$^1$H ($\delta$ = ppm)
$\delta$ = 7,35 ppm : massif : aromatiques.

**EXEMPLE 16 : METHYLOXYIMINO (E + Z) - 9 (S) BENZYLAMINO - 20 CYANO - 20 DIDESOXO - 9,20 TYLOSINE**

En procédant comme dans l'exemple 15, mais en remplaçant au stade A la (R) benzylamino - 20 cyano - 20 désoxo - 20 tylosine par la (S) benzylamino - 20 cyano - 20 désoxo - 20 tylosine, on obtient le produit attendu.

**EXEMPLES 17 ET 18 : PARANITROBENZYLOXYIMINO (E + Z) - 9 (R) BENZYLAMINO - 20 CYANO - 20 DIDESOXO - 9,20 TYLOSINE**

ET

**PARANITROBENZYLOXYIMINO (E + Z) - 9 (S) BENZYLAMINO - 20 CYANO - 20 DIDESOXO - 9,20 TYLOSINE**

En remplaçant dans les exemples 15 et 16 le chlorhydrate de méthoxyamine par le chlorhydrate de paranitrobenzyloxyamine, on obtient les produits attendus.

**EXEMPLE 19 : BENZYLOXYIMINO (E + Z) - 9 DIBENZYLAMINO - 20 CYANO -20 DIDESOXO - 9,20 DEMYCAROSYL DEMYCINOSYL TYLOSINE**

**STADE A : DEMYCAROSYL TYLOSINE**

Agiter 4 g (0,004 mmole) de tylosine base dans 80 ml d'acide chlorhydrique 0,2 N pendant 4 heures à la température ambiante. Laver le milieu réactionnel obtenu au dichlorométhane, séparer la phase aqueuse et l'ajuster à PH 8,0. Extraire à deux reprises par 120 ml de dichlorométhane, réunir les phases organiques, sécher sur sulfate de sodium et évaporer. Le résidu est constitué de démycarosyl tylosine.
Rendement : 94 %
Caractéristiques spectrales :
Spectrométrie de masse :
$[M - H]^+$ : M/Z : 772

**STADE B : DEMYCAROSYL DEMYCINOSYL TYLOSINE**

Dissoudre 5 g (0,0065 mmole) de démycarosyl tylosine obtenu au stade A dans 110 ml d'acide chlorhydrique 0,5 N et agiter pendant 27 heures environ à 75 °C. Laver le milieu réactionnel au dichlorométhane. Récupérer la phase aqueuse, l'ajuster à PH 8 et extraire à deux reprises par 120 ml de dichlorométhane. Sécher la phase organique sur sulfate de sodium et évaporer. Le résidu est chromatographié sur gel de silice (éluant $CH_2Cl_2$ ; MEOH ; $NH_4OH$ ; 10 ; 1 ; 0,05) pour obtenir le produit attendu.

Rendement : 20 %

Caractéristiques spectrales :

Spectrométrie de masse :

$[M - H]^+$ : M/Z : 598

**STADE C : BENZYLOXYIMINO (E + Z) - 9 DIBENZYLAMINO - 20 CYANO - 20 DIDESOXO - 9,20 DEMYCAROSYL DEMYCINOSYL TYLOSINE**

En procédant comme dans l'exemple 2, mais en remplaçant la tylosine par la démycarosyl démycinosyl tylosine, on obtient le produit attendu.

**EXEMPLE 20 : N BENZYL NITRONE EN 20 DE LA BENZYLOXYIMINO (E + Z) -9 DIDESOXO - 9,20 DEMYCAROSYL DEMYCINOSYL TYLOSINE**

En procédant comme dans l'exemple 9, mais en remplaçant au stade A la tylosine par la démycarosyl démycinosyl tylosine obtenue au stade B de l'exemple 19, on obtient le produit attendu.

**EXEMPLE 21 : ETUDE DE L'ACTIVITE DES PRODUITS DE L'INVENTION SUR DIVERSES SOUCHES BACTERIENNES**

La détermination des concentrations minimales inhibitrices (CMI) est effectuée :

- pour les Staphylocoques et les Entérocoques (Streptocoques D) en milieu gélosé ou liquide de MUELLER HINTON ;
- pour les Hémophilus, Streptocoques non D et Neisseria Gonorrhoeae, la détermination des CMI est effectuée suivant la méthode de dilution en milieu gélosé au sang cuit enrichi du mélange Polyvitex*. La culture est faite en atmosphère enrichie en $CO_2$.

La lecture des CMI est effectuée après 18 heures d'incubation à 37 °C.

Les produits sont testés dans la gamme de concentrations de 0,125 à 256 mg/l (dilutions successives de raison 2).

Les concentrations minimales inhibitrices sont de l'ordre :

- du mg. $l^{-1}$ pour le Staphylocoque doré, les Streptocoques B, C, D, G les Pneumocoques ;
- de 0,5 mg. $l^{-1}$ pour les Streptocoques A ;
- de 0,1 mg. $l^{-1}$ pour Neisseria Gonorrhoeae.

L'ensemble de ces études montre l'activité antibiotique intéressante du produit de l'invention à la fois par l'intensité de son activité ainsi que par l'étendue de son spectre d'action.

**EXEMPLE 22 : COMPOSITION PHARMACEUTIQUE : COMPRIME**

Comprimés dosés à 100 mg de benzyloxyimino (E + Z) - 9 dibenzylamino - 20 cyano - 20 didésoxo 9,20 tylosine.

| | |
|---|---|
| Benzyloxyimino (E + Z) - 9 dibenzylamino - 20 cyano - 20 didésoxo - 9,20 tylosine ..... | 100 g |
| Amidon de blé ..... | 70 g |
| Amidon de maïs ..... | 60 g |
| Lactose ..... | 60 g |
| Stéarate de magnésium ..... | 9 g |
| Silice ..... | 4 g |
| Hydroxy propylcellulose ..... | 7 g |
| Formule de préparation pour 1000 comprimés, | |

## Revendications

1/ Composés de formule (I) :

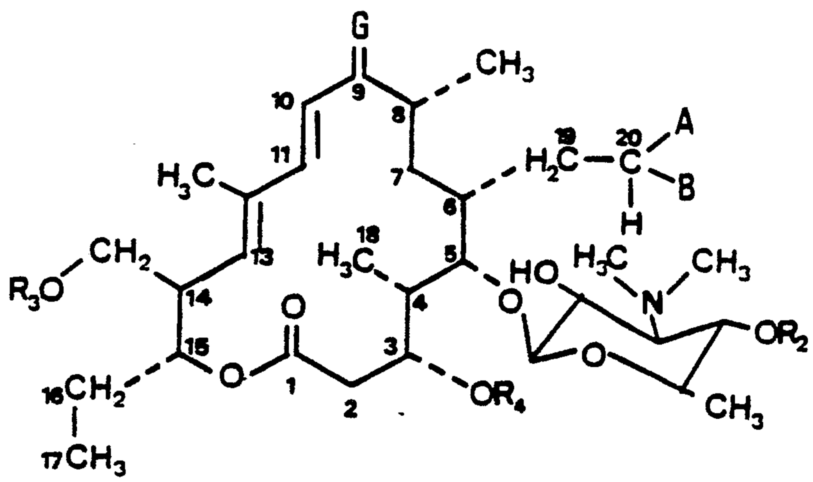

(I)

dans laquelle :
ou bien :
A représente un groupement

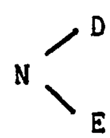

dans lequel D et E identiques ou différents représentent indépendamment l'un de l'autre un atome d'hydrogène, un radical alcoyle inférieur linéaire ou ramifié, éventuellement substitué par un groupement phényle et dans ce cas B représente un groupement cyano,
ou bien :
A et B représentent ensemble un groupement = $N^{+}$ ($O^{-}$) - D dans lequel D a la même signification que précédemment,
G représente un atome d'oxygène ou un groupement de formule N ∿ O -$R_1$ dans lequel $R_1$ représente un atome d'hydrogène, un groupement alcoyle inférieur linéaire ou ramifié, un groupement alkényle inférieur linéaire ou ramifié, ceux-ci étant optionnellement substitué par un groupement phényle lui-même éventuellement substitué par un groupement nitro,
le signe ∿ existant dans la définition de G signifiant que la fonction oxime ou éther d'oxime peut se trouver sous la forme syn ou anti ou sous forme du mélange syn et anti,
$R_2$ représente . soit un atome d'hydrogène,
. soit un radical de formule :

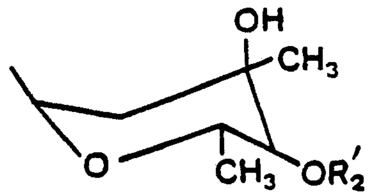

dans lequel $R'_2$ représente un atome d'hydrogène, un radical alcoyle inférieur linéaire ou ramifié ou un radical acyle inférieur, linéaire ou ramifié,

$R_3$ représente un atome d'hydrogène ou un groupement :

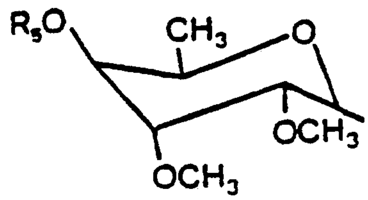

$R_4$ et $R_5$ représentent indépendamment l'un de l'autre :

. soit un atome d'hydrogène,

. soit un radical alcoyle inférieur linéaire ou ramifié,

étant entendu que par radicaux alcoyle inférieur et alkényle inférieur, on entend des groupements comprenant entre 1 et 6 atomes de carbone,

leurs isomères, diastéréoisomères ainsi que leurs sels d'addition à un acide pharmaceutiquement acceptable.

2/ Composés selon la revendication 1, dans laquelle A représente un groupement NDE et B représente un groupement cyano, leurs isomères, diastéréoisomères, ainsi que leurs sels d'addition à un acide pharmaceutiquement acceptable.

3/ Composés selon la revendication 1 caractérisés en ce que A et B représentent simultanément un groupement = $N^+$ ($O^-$) - D, leurs isomères, diastéréoisomères, ainsi que leurs sels d'addition à un acide pharmaceutiquement acceptable.

4/ Composés selon la revendication 1, caractérisés en ce que G représente un atome d'oxygène, leurs isomères, diastéréoisomères, ainsi que leurs sels d'addition à un acide pharmaceutiquement acceptable.

5/ Composés selon la revendication 1, caractérisés en ce que G représente un groupement N ∿ $OR_1$, leurs isomères, diastéréoisomères, ainsi que leurs sels d'addition à un acide pharmaceutiquement acceptable.

6/ Composés selon l'une des revendications 1 ou 2 qui est la benzyloxyimino (E + Z) - 9 dibenzylamino - 20 cyano - 20 didesoxo - 9,20 tylosine.

7/ Composé selon l'une des revendications 1 ou 3 qui est la N benzyl nitrone en 20 de la benzyloxyimino - 9 (E + Z) didesoxo - 9,20 tylosine.

8/ Composé selon l'une des revendications 1 ou 2 qui est la para nitro benzyloxyimino (E + Z) - 9 (R, S) benzylamino - 20 cyano - 20 didesoxo - 9,20 tylosine, ainsi que chacun de ses isomères R et S.

9/ Procédé de préparation de composés de formule (I) selon la revendication 1 caractérisé en ce que l'on utilise comme matière première un dérivé de formule (II) :

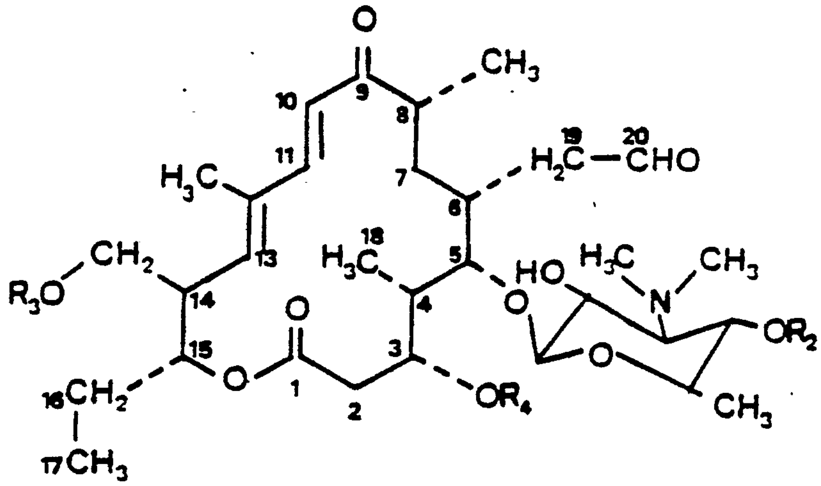

(II)

dans laquelle $R_2$, $R_3$, $R_4$ ont la même définition que dans la formule (I), qui, dans le cas où dans le produit de formule (I) que l'on souhaite obtenir $R_2$ représente un atome d'hydrogène, est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

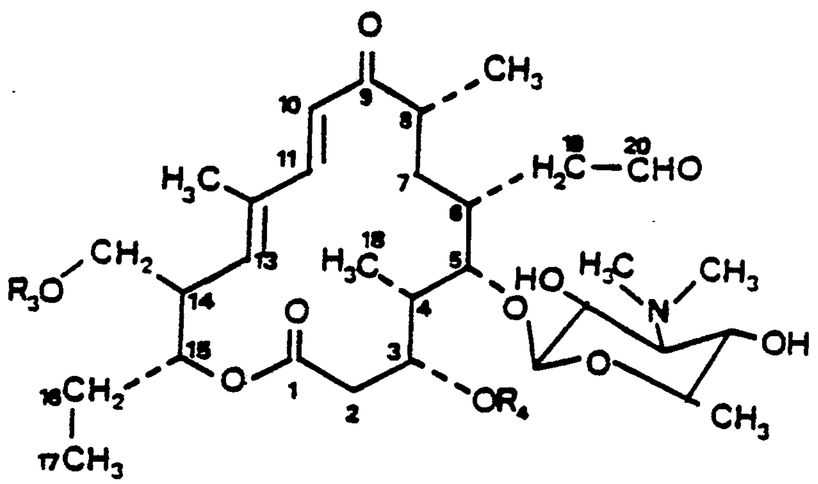

(II/2a)

dans laquelle $R_3$ et $R_4$ ont la même signification que dans la formule (I), qui, dans le cas où dans le produit de formule (I) que l'on souhaite obtenir $R_2$ et $R_3$ simultanément représentent un atome d'hydrogène, est ensuite soumis à réaction avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100 °C, préférentiellement comprise entre 50 et 90 °C, préférentiellement comprise entre 70 et 80 °C, pour conduire, après lavage par un solvant organique approprié, alcalinisation et extraction par un solvant organique approprié à un dérivé de formule (II/2b) :

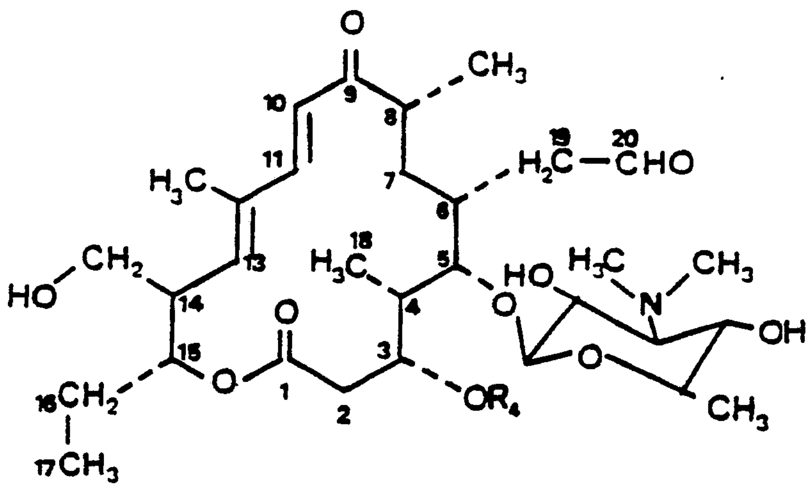

(II/2b)

dans laquelle :
$R_4$ a la même signification que dans la formule (I), que l'on purifie éventuellement par chromatographie sur colonne de silice,
le dérivé alors choisi de formule générale (II), ou (II/2a), ou (II/2b) selon le composé de formule (I) que l'on souhaite obtenir étant alors traité :
- soit, dans le cas où B représente un groupement cyano, par un cyano phosphonate de dialkyle tel que le cyano phosphonate de diéthyle,
en présence d'une amine de formule (III) HNDE où D et E ont la même signification que dans la formule (I) dans un solvant organique, préférentiellement choisi parmi tétrahydrofuranne, éther diéthylique, éther diisopropylique, benzène, dioxanne, acétone, acétate d'éthyle,
à une température comprise inclusivement entre la température ambiante et la température du reflux du solvant choisi pour conduire à un dérivé de formule (I/A), cas particulier des dérivés de formule (I) pour lesquels G représente un atome d'oxygène, A un groupement NDE et B un groupement cyano,

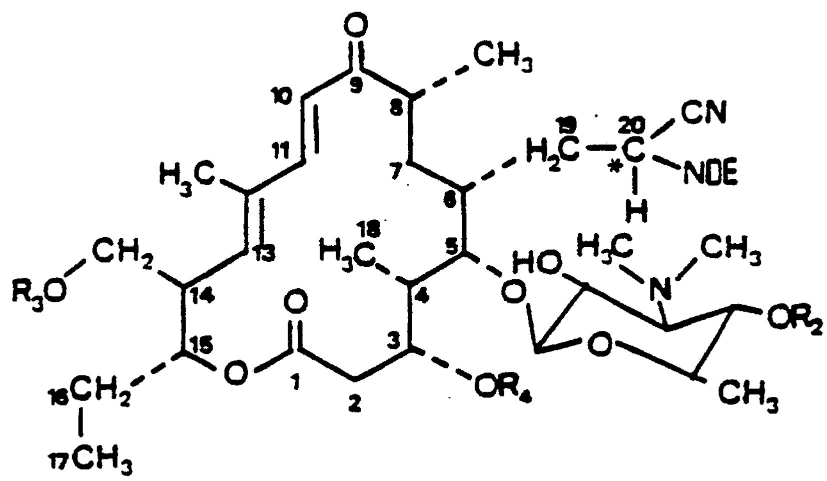

(I/A)

dans laquelle :
D, E et $R_4$ ont la même définition que dans la formule (I), et $R_2$ et $R_3$ la même signification que dans le dérivé choisi (II), (II/2a) ou (II/2b) dont on sépare, si on le désire, les isomères (R) et (S) par une technique classique telle que la chromatographie sur colonne de silice ou la cristallisation fractionnée,
- soit dans le cas où A et B simultanément représentent un groupement = $N^+$ ($O^-$) - D par un dérivé de formule HO - NHD dans lequel D a la même définition que dans la formule (I), ou bien préférentiellement avec un sel d'acide fort d'un tel produit (chlorhydrate, bromhydrate...) dans un solvant organique préférentiellement choisi parmi alcools aliphatiques inférieurs, acétate d'éthyle, acétonitrile en présence d'une base comme par exemple la pyridine, la triéthylamine ou un sel de métal alcalin tel que l'acétate de sodium ou le carbonate acide de sodium ou le carbonate acide de potassium ou le carbonate de sodium, ou le

carbonate de potassium ou le carbonate de calcium pour conduire à un dérivé de formule (I/B) :

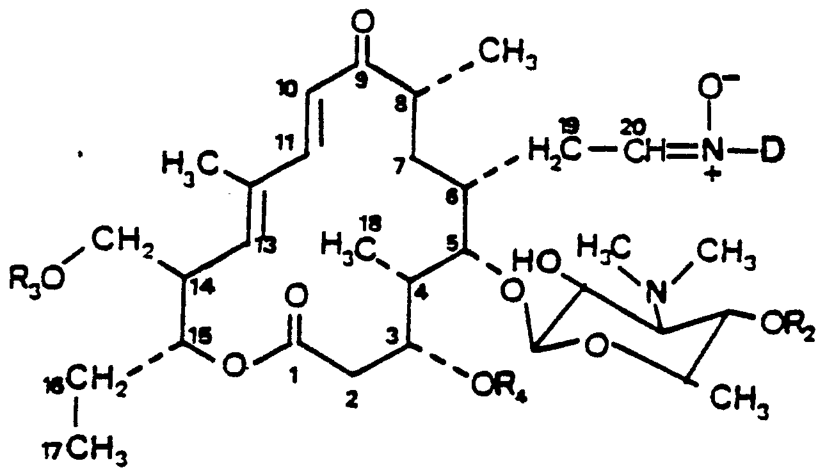

(I/B)

cas particulier des dérivés de formule (I) dans lequel G représente un atome d'oxygène, et A et B représentent simultanément un groupement = $N^{+}$ -($O^{-}$) - D,
dans laquelle $R_4$ et D ont la même signification que dans la formule (I) et $R_2$ et $R_3$ la même signification que dans la formule (II), (II/2a) ou (II/2b) du dérivé choisi,
dérivé de formule (I/A) ou (I/B) que l'on traite si on le désire en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium avec un dérivé de formule (V) :
**$H_2N$ - O - $R_1$ (V)**
dans lequel $R_1$ a la même signification que dans la formule (I) ou bien préférentiellement avec un sel d'acide fort (chlorhydrate, bromhydrate...) d'un tel produit pour obtenir après purification éventuelle par chromatographie sur colonne de silice un dérivé de formule (I/D) :

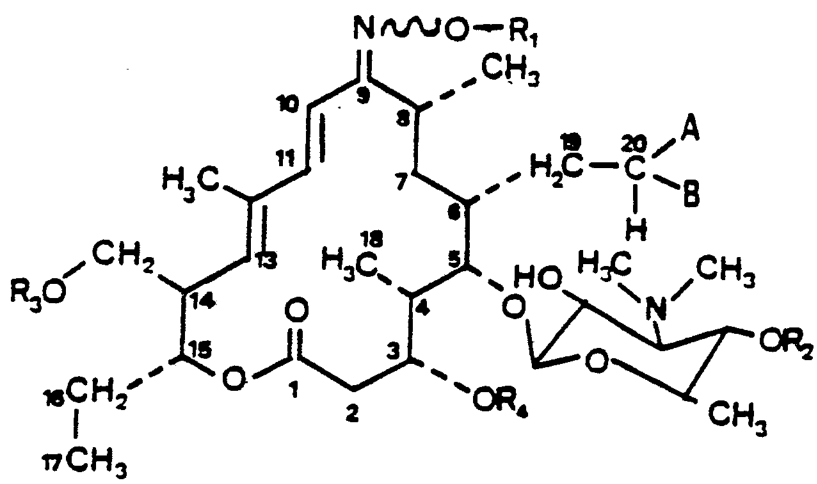

(I/D)

cas particulier des dérivés de formule (I) dans lesquels G représente un groupement N O - $R_1$ et A, B et $R_4$ ont la même signification que dans le dérivé de formules (I/A) ou (I/B),
- que l'on purifie éventuellement par chromatographie sur colonne de silice à l'aide d'un mélange solvant approprié comme par exemple un mélange chlorure de méthylène/méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

10/ Procédé de préparation de composés de formule (I), selon l'une des revendications 1 ou 2 caractérisé en ce que l'on utilise comme matière première un dérivé de formule (II) :

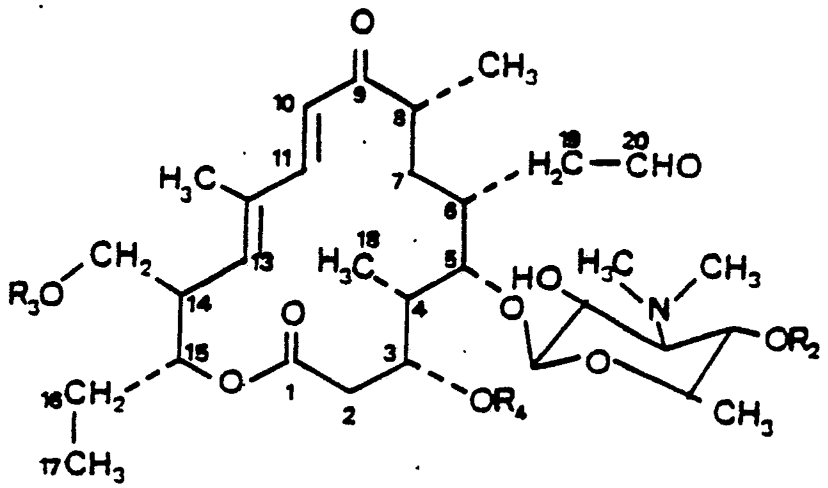

(II)

dans laquelle $R_2$, $R_3$, $R_4$ ont la même définition que dans la formule (I), qui, dans le cas où dans le produit de formule (I) que l'on souhaite obtenir $R_2$ représente un atome d'hydrogène, est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

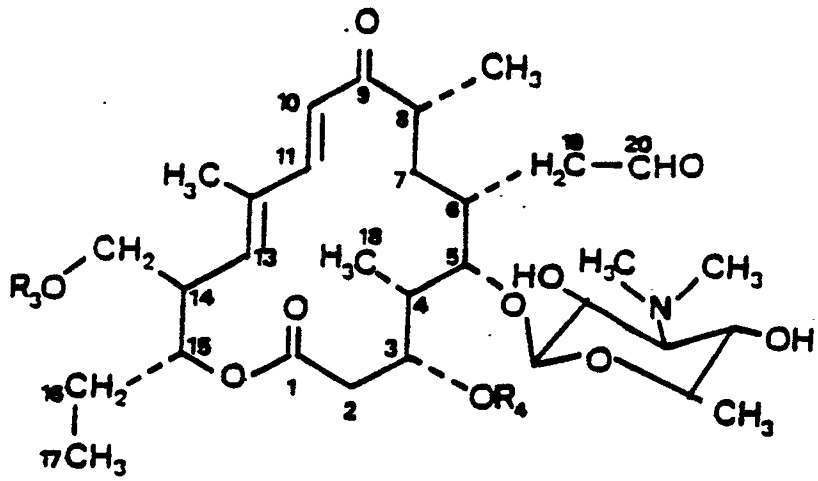

(II/2a)

dans laquelle $R_3$ et $R_4$ ont la même signification que dans la formule (I), qui, dans le cas où dans le produit de formule (I) que l'on souhaite obtenir $R_2$ et $R_3$ simultanément représentent un atome d'hydrogène, est ensuite soumis à réaction avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100 °C, préférentiellement comprise entre 50 et 90 °C, préférentiellement comprise entre 70 et 80 °C, pour conduire, après lavage par un solvant organique approprié, alcalinisation et extraction par un solvant organique approprié à un dérivé de formule (II/2b) :

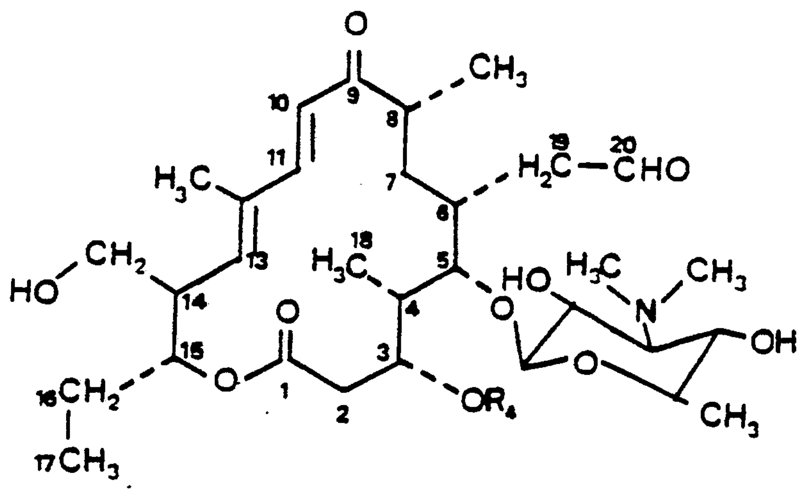

(II/2b)

dans laquelle :
$R_4$ a la même signification que dans la formule (I), que l'on purifie éventuellement par chromatographie sur colonne de silice,
le dérivé alors choisi de formule générale (II), ou (II/2a), ou (II/2b) selon le composé de formule (I) que l'on souhaite obtenir étant alors traité par un cyano phosphonate de dialkyle tel que le cyano phosphonate de diéthyle,
en présence d'une amine de formule (III) HNDE où D et E ont la même signification que dans la formule (I) dans un solvant organique, préférentiellement choisi parmi tétrahydrofuranne, éther diéthylique, éther diisopropylique, benzène, dioxanne, acétone, acétate d'éthyle,
à une température comprise inclusivement entre la température ambiante et la température du reflux du solvant choisi pour conduire à un dérivé de formule (I/A), cas particulier des dérivés de formule (I) pour lesquels G représente un atome d'oxygène, A un groupement NDE et B un groupement cyano,

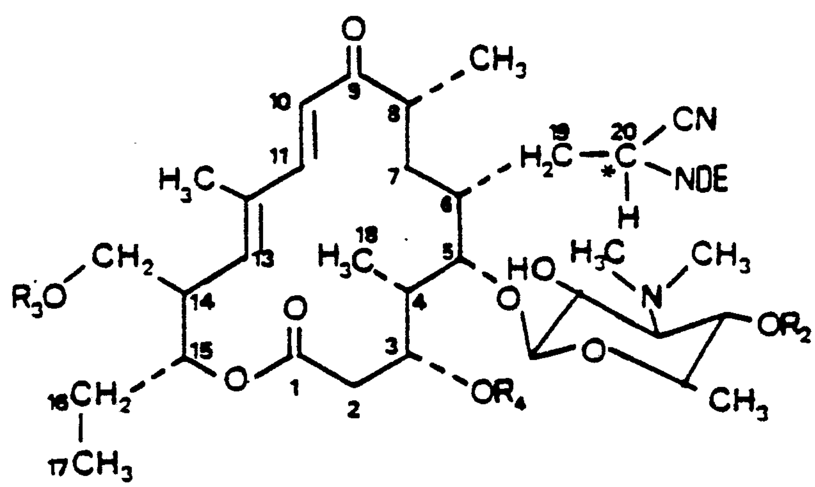

(I/A)

dans laquelle :
D, E et $R_4$ ont la même définition que dans la formule (I), et $R_2$ et $R_3$ la même signification que dans le dérivé choisi (II), (II/1a) ou (II/2b) dont on sépare, si on le désire, les isomères (R) et (S) par une technique classique telle que la chromatographie sur colonne de silice ou la cristallisation fractionnée,
dérivé de formule (I/A) que l'on traite si on le désire en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium avec un dérivé de formule (V) :
**$H_2N$ - O - $R_1$ (V)**
dans lequel $R_1$ a la même signification que dans la formule (I) ou bien préférentiellement avec un sel d'acide fort (chlorhydrate, bromhydrate...) d'un tel produit pour obtenir après purification éventuelle par

chromatographie sur colonne de silice un dérivé de formule (I/D1) :

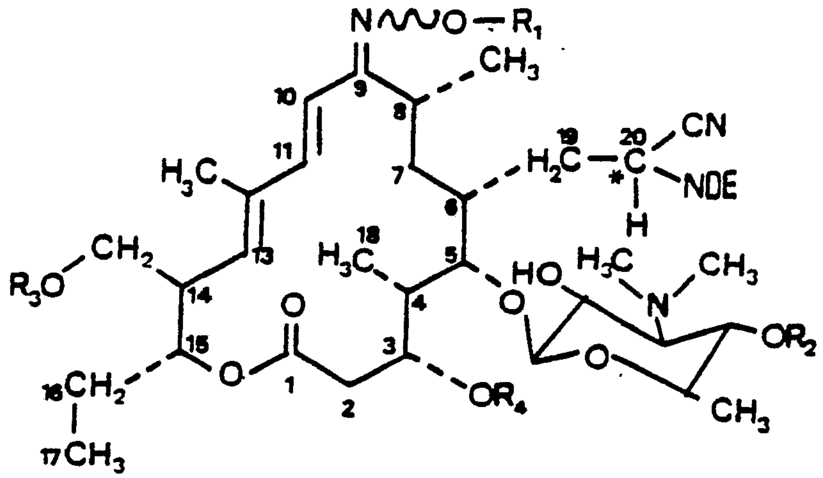

(I/D1)

cas particulier des dérivés de formule (I) dans lesquels G représente un groupement N ∿ O - $R_1$ et D, E et $R_4$ ont la même signification que dans la formule du dérivé (I/A),
- que l'on purifie éventuellement par chromatographie sur colonne de silice à l'aide d'un mélange solvant approprié comme par exemple un mélange chlorure de méthylène/méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

11/ Procédé de préparation de composés de formule (I), selon l'une des revendications 1 ou 3 caractérisé en ce que l'on utilise comme matière un dérivé de formule (II) :

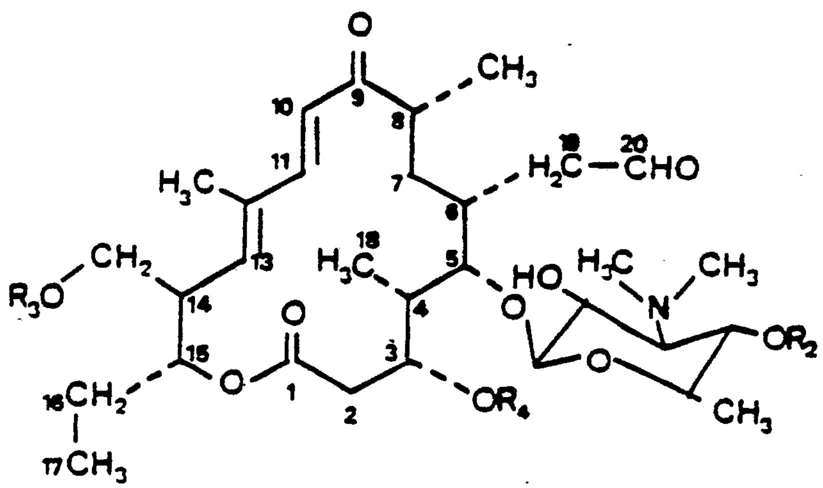

(II)

dans laquelle $R_2$, $R_3$, $R_4$ ont la même définition que dans la formule (I),
qui, dans le cas où dans le produit de formule (I) que l'on souhaite obtenir $R_2$ représente un atome d'hydrogène, est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

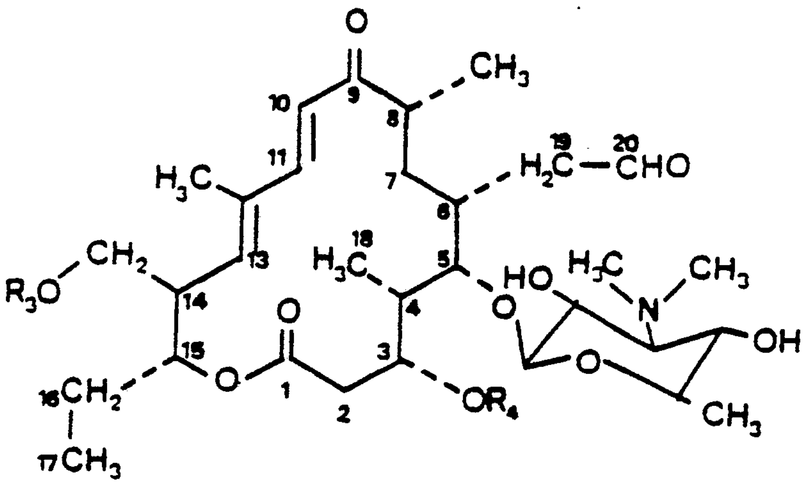

(II/2a)

dans laquelle $R_3$ et $R_4$ ont la même signification que dans la formule (I), qui, dans le cas où dans le produit de formule (I) que l'on souhaite obtenir $R_2$ et $R_3$ simultanément représentent un atome d'hydrogène, est ensuite soumis à réaction avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100 °C, préférentiellement comprise entre 50 et 90 °C, préférentiellement comprise entre 70 et 80 °C, pour conduire, après lavage par un solvant organique approprié, alcalinisation et extraction par un solvant organique approprié à un dérivé de formule (II/2b) :

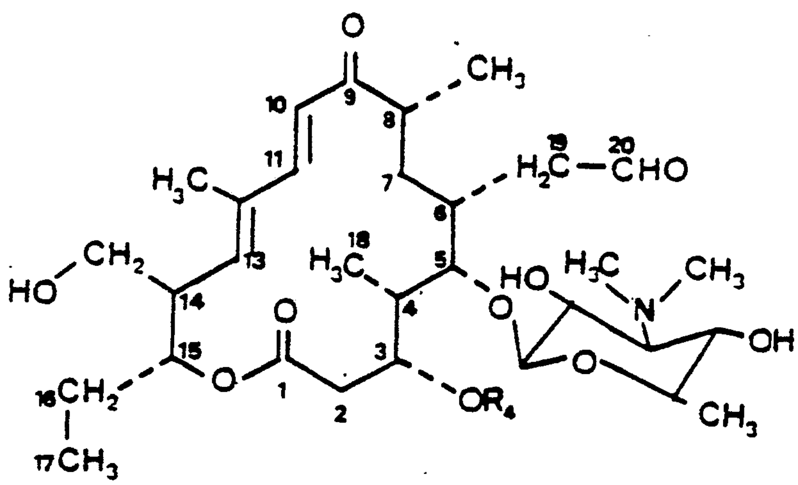

(II/2b)

dans laquelle :

$R_4$ a la même signification que dans la formule (I), que l'on purifie éventuellement par chromatographie sur colonne de silice,

le dérivé alors choisi de formule générale (II), ou (II/2a), ou (II/2b) selon le composé de formule (I) que l'on souhaite obtenir étant alors traité par un dérivé de formule HO - NHD dans lequel D a la même définition que dans la formule (I), ou bien préférentiellement avec un sel d'acide fort d'un tel produit (chlorhydrate, bromhydrate...) dans un solvant organique préférentiellement choisi parmi alcools aliphatiques inférieurs, acétate d'éthyle, acétonitrile en présence d'une base comme par exemple la pyridine, la triéthylamine ou un sel de métal alcalin tel que l'acétate de sodium ou le carbonate acide de sodium ou le carbonate acide de potassium ou le carbonate de sodium, ou le carbonate de potassium ou le carbonate de calcium pour conduire à un dérivé de formule (I/B) :

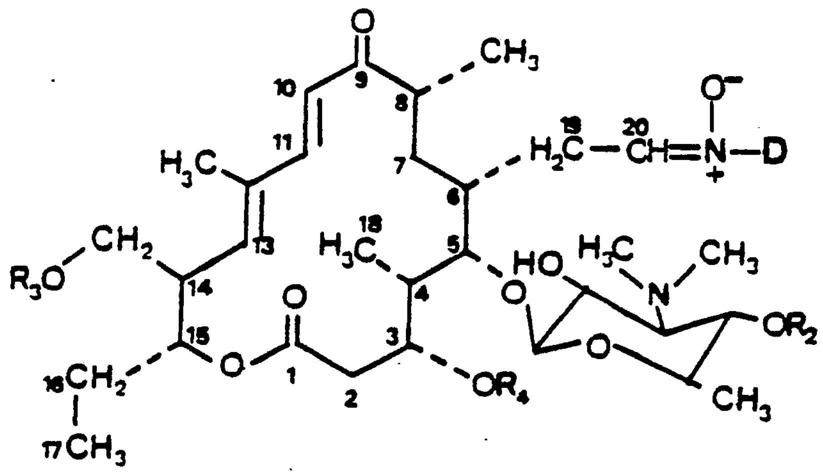

(I/B)

cas particulier des dérivés de formule (I) dans lequel G représente un atome d'oxygène, et A et B représentent simultanément un groupement = $N^{+}$ -(O⁻) - D,
dans laquelle $R_4$ et D ont la même signification que dans la formule (I) et $R_2$ et $R_3$ la même signification que dans la formule (II), (II/2a) ou (II/2b) du dérivé choisi,
dérivé de formule (I/B) que l'on traite si on le désire en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium avec un dérivé de formule (V) :

**$H_2N$ - O - $R_1$ (V)**

dans lequel $R_1$ a la même signification que dans la formule (I) ou bien préférentiellement avec un sel d'acide fort (chlorhydrate, bromhydrate...) d'un tel produit pour obtenir après purification éventuelle par chromatographie sur colonne de silice un dérivé de formule (I/D2) :

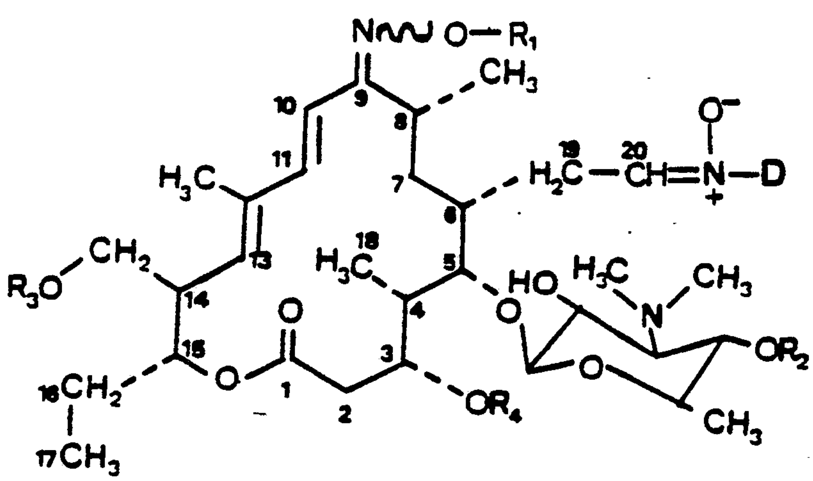

(I/D2)

cas particulier des dérivés de formule (I) dans lesquels G représente un groupement N ∿ O - $R_1$ et A, B et $R_4$ ont la même signification que dans la formule du dérivé (I/B),
- que l'on purifie éventuellement par chromatographie sur colonne de silice à l'aide d'un mélange solvant approprié comme par exemple un mélange chlorure de méthylène/méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

12/ Composition pharmaceutique contenant comme principe actif au moins un composés selon l'une des revendications 1 à 8 en combinaison avec un ou plusieurs excipients ou véhicules inertes, non toxiques, pharmaceutiquement acceptables.

13/ Compositions pharmaceutiques selon la revendication 12 contenant au moins un principe actif selon l'une des revendications 1 à 8 utilisables dans le traitement des maladies justifiant une antibiothérapie à

large spectre.

Revendications pour l'Etat contractant suivant: ES

1/ Procédé de préparation de composés de formule (I) :

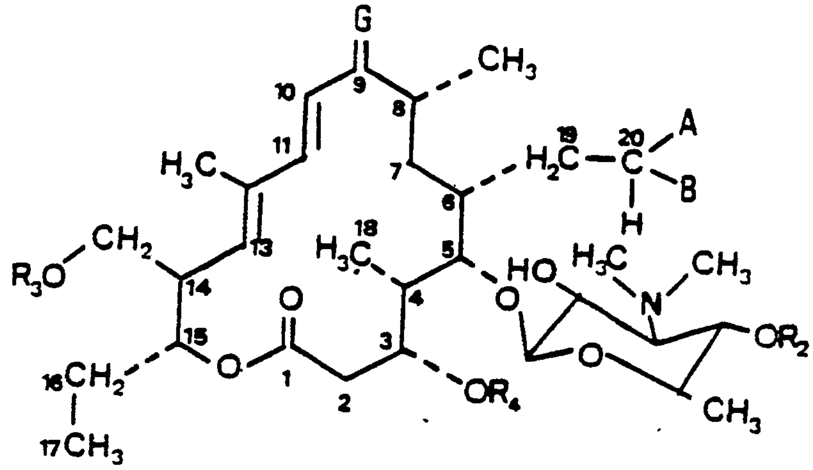

(I)

dans laquelle :
ou bien :
A représente un groupement

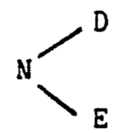

dans lequel D et E identiques ou différents représentent indépendamment l'un de l'autre un atome d'hydrogène, un radical alcoyle inférieur linéaire ou ramifié, éventuellement substitué par un groupement phényle et dans ce cas B représente un groupement cyano,
ou bien :
A et B représentent ensemble un groupement = $N^{+}$ ($O^{-}$) - D dans lequel D a la même signification que précédemment,
G représente un atome d'oxygène ou un groupement de formule N ∿ O -$R_1$ dans lequel $R_1$ représente un atome d'hydrogène, un groupement alcoyle inférieur linéaire ou ramifié, un groupement alkényle inférieur linéaire ou ramifié, ceux-ci étant optionnellement substitué par un groupement phényle lui-même éventuellement substitué par un groupement nitro,
le signe ∿ existant dans la définition de G signifiant que la fonction oxime ou éther d'oxime peut se trouver sous la forme syn ou anti ou sous forme du mélange syn et anti,
$R_2$ représente . soit un atome d'hydrogène,
. soit un radical de formule :

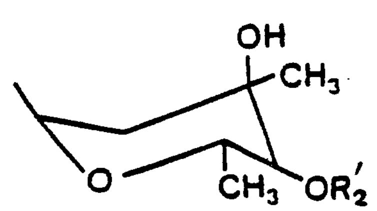

dans lequel $R'_2$ représente un atome d'hydrogène, un radical alcoyle inférieur linéaire ou ramifié ou un radical acyle inférieur, linéaire ou ramifié,
$R_3$ représente un atome d'hydrogène ou un groupement :

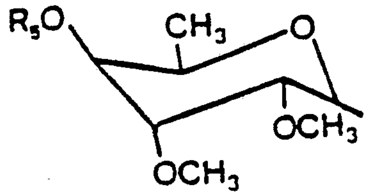

$R_4$ et $R_5$ représentent indépendamment l'un de l'autre :
. soit un atome d'hydrogène,
. soit un radical alcoyle inférieur linéaire ou ramifié,
étant entendu que par radicaux alcoyle inférieur et alkényle inférieur, on entend des groupements comprenant entre 1 et 6 atomes de carbone,
leurs isomères, diastéréoisomères ainsi que leurs sels d'addition à un acide pharmaceutiquement acceptable,
procédé caractérisé en ce que l'on utilise comme matière première un dérivé de formule (II) :

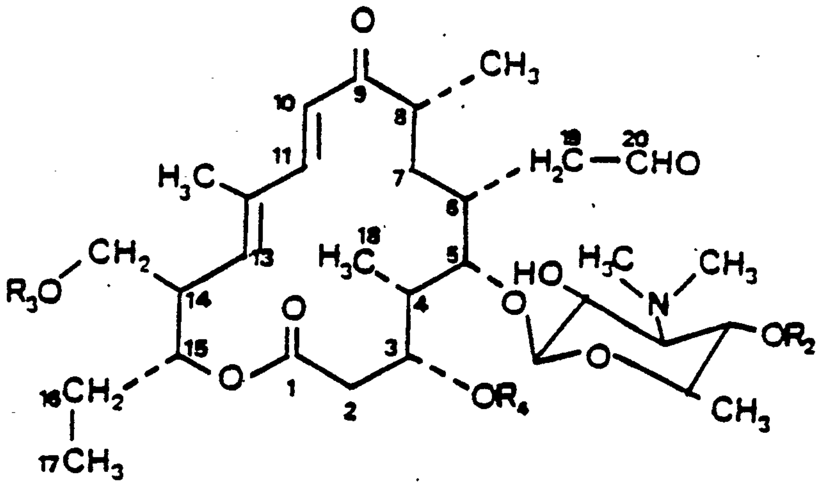

(II)

dans laquelle $R_2$, $R_3$, $R_4$ ont la même définition que dans la formule (I),
qui, dans le cas où dans le produit de formule (I) que l'on souhaite obtenir $R_2$ représente un atome d'hydrogène, est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

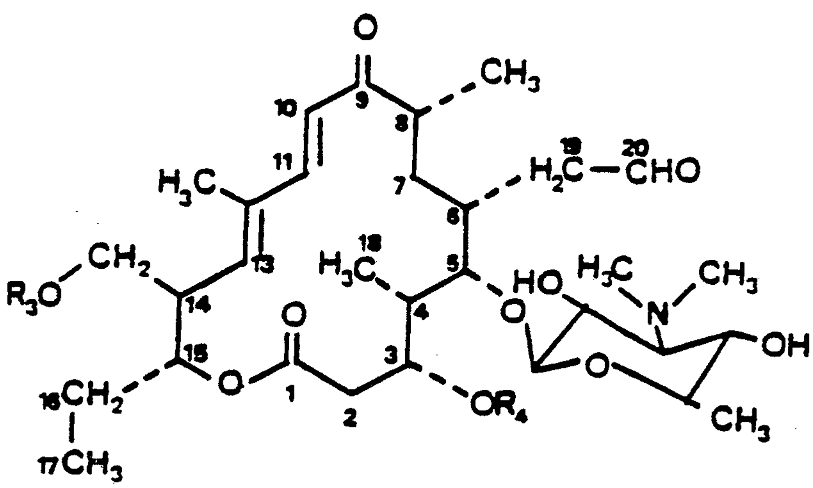

(II/2a)

dans laquelle $R_3$ et $R_4$ ont la même signification que dans la formule (I), qui, dans le cas où dans le produit de formule (I) que l'on souhaite obtenir $R_2$ et $R_3$ simultanément représentent un atome d'hydrogène, est ensuite soumis à réaction avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100° C, préférentiellement comprise entre 50 et 90° C, préférentiellement comprise entre 70 et 80° C, pour conduire, après lavage par un solvant organique approprié, alcalinisation et extraction par un solvant organique approprié à un dérivé de formule (II/2b) :

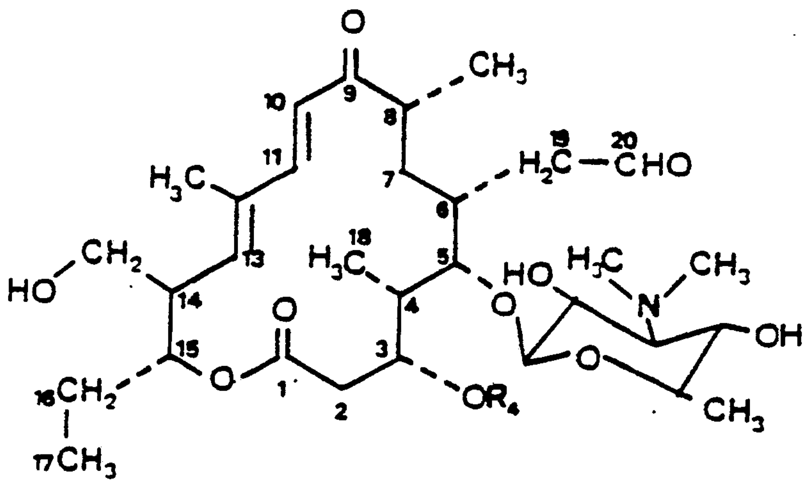

(II/2b)

dans laquelle :

$R_4$ a la même signification que dans la formule (I), que l'on purifie éventuellement par chromatographie sur colonne de silice,

le dérivé alors choisi de formule générale (II), ou (II/2a), ou (II/2b) selon le composé de formule (I) que l'on souhaite obtenir étant alors traité :

- soit, dans le cas où B représente un groupement cyano, par un cyano phosphonate de dialkyle tel que le cyano phosphonate de diéthyle,

en présence d'une amine de formule (III) HNDE où D et E ont la même signification que dans la formule (I) dans un solvant organique, préférentiellement choisi parmi tétrahydrofuranne, éther diéthylique, éther diisopropylique, benzène, dioxanne, acétone, acétate d'éthyle,

à une température comprise inclusivement entre la température ambiante et la température du reflux du solvant choisi pour conduire à un dérivé de formule (I/A), cas particulier des dérivés de formule (I) pour lesquels G représente un atome d'oxygène, A un groupement NDE et B un groupement cyano,

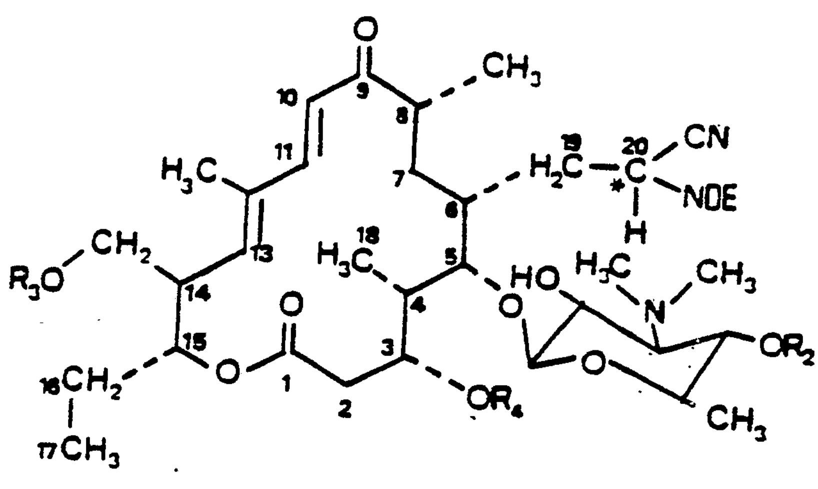

(I/A)

dans laquelle :

D, E et $R_4$ ont la même définition que dans la formule (I), et $R_2$ et $R_3$ la même signification que dans le

dérivé choisi (II), (II/2a) ou (II/2b) dont on sépare, si on le désire, les isomères (R) et (S) par une technique classique telle que la chromatographie sur colonne de silice ou la cristallisation fractionnée,

- soit dans le cas où A et B simultanément représentent un groupement = $N^+$ ($O^-$) - D par un dérivé de formule HO - NHD dans lequel D a la même définition que dans la formule (I), ou bien préférentiellement avec un sel d'acide fort d'un tel produit (chlorhydrate, bromhydrate...) dans un solvant organique préférentiellement choisi parmi alcools aliphatiques inférieurs, acétate d'éthyle, acétonitrile en présence d'une base comme par exemple la pyridine, la triéthylamine ou un sel de métal alcalin tel que l'acétate de sodium ou le carbonate acide de sodium ou le carbonate acide de potassium ou le carbonate de sodium, ou le carbonate de potassium ou le carbonate de calcium pour conduire à un dérivé de formule (I/B) :

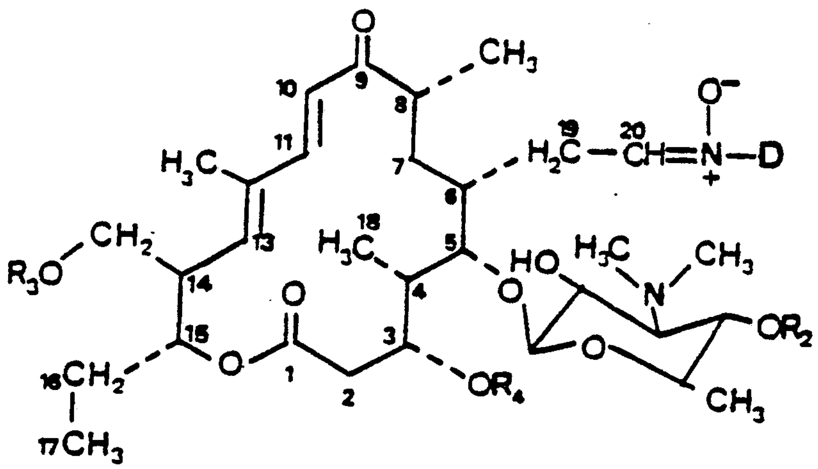

(I/B)

cas particulier des dérivés de formule (I) dans lequel G représente un atome d'oxygène, et A et B représentent simultanément un groupement = $N^+$ -($O^-$) - D,
dans laquelle $R_4$ et D ont la même signification que dans la formule (I) et $R_2$ et $R_3$ la même signification que dans la formule (II), (II/2a) ou (II/2b) du dérivé choisi,
dérivé de formule (I/A) ou (I/B) que l'on traite si on le désire en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium avec un dérivé de formule (V) :

**$H_2N$ - O - $R_1$ (V)**

dans lequel $R_1$ a la même signification que dans la formule (I) ou bien préférentiellement avec un sel d'acide fort (chlorhydrate, bromhydrate...) d'un tel produit pour obtenir après purification éventuelle par chromatographie sur colonne de silice un dérivé de formule (I/D) :

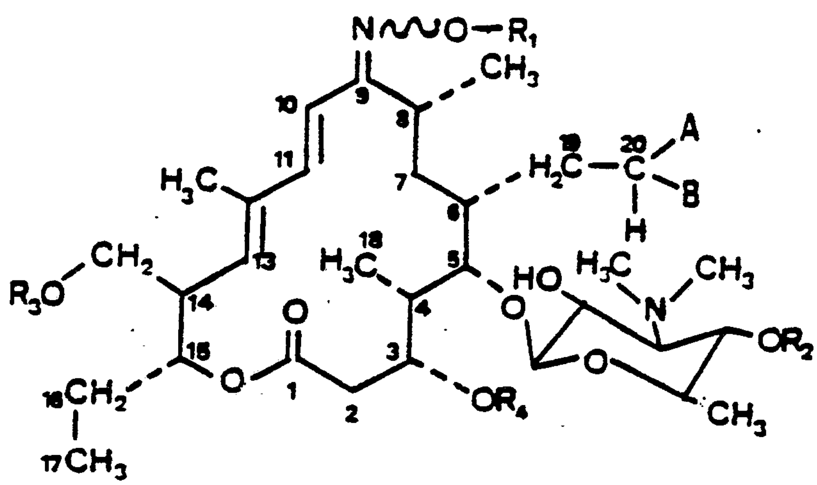

(I/D)

cas particulier des dérivés de formule (I) dans lesquels G représente un groupement N O - $R_1$ et A, B et $R_4$ ont la même signification que dans le dérivé de formules (I/A) ou (I/B),

- que l'on purifie éventuellement par chromatographie sur colonne de silice à l'aide d'un mélange solvant approprié comme par exemple un mélange chlorure de méthylène/méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

2/ Procédé de préparation de composés de formule (I), selon la revendication 1, caractérisé en ce que l'on utilise comme matière première un dérivé de formule (II) :

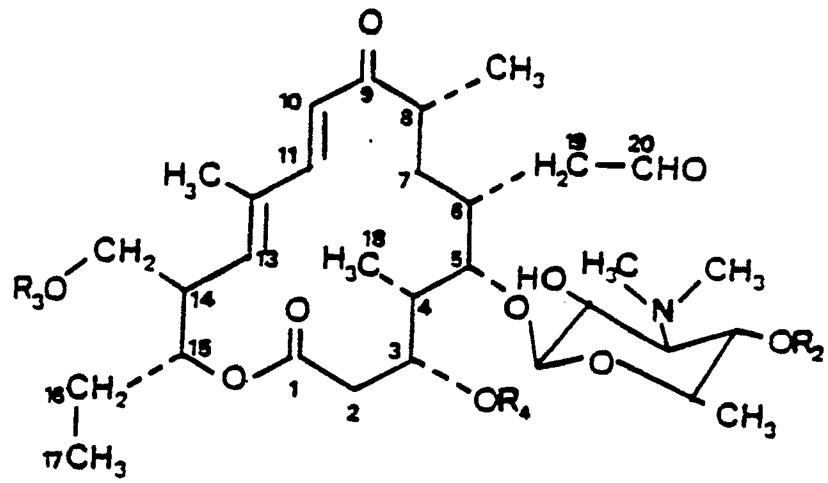

(II)

dans laquelle $R_2$, $R_3$, $R_4$ ont la même définition que dans la formule (I),
qui, dans le cas où dans le produit de formule (I) que l'on souhaite obtenir $R_2$ représente un atome d'hydrogène, est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

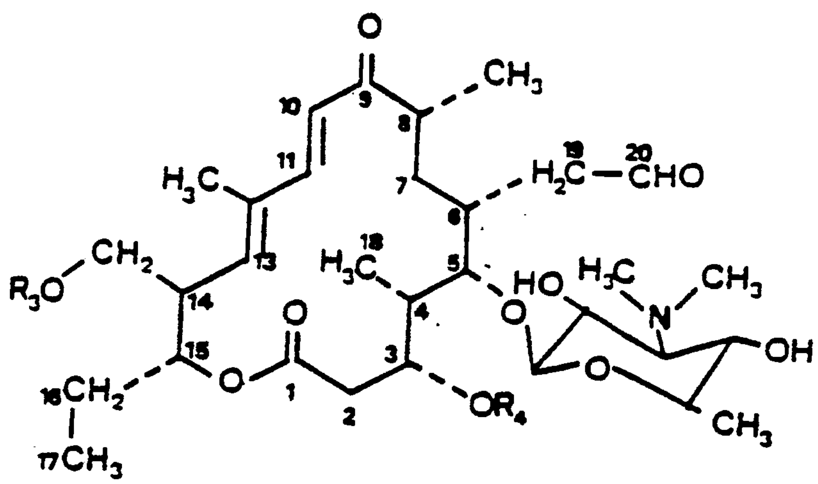

(II/2a)

dans laquelle $R_3$ et $R_4$ ont la même signification que dans la formule (I),
qui, dans le cas où dans le produit de formule (I) que l'on souhaite obtenir $R_2$ et $R_3$ simultanément représentent un atome d'hydrogène, est ensuite soumis à réaction avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100 °C, préférentiellement comprise entre 50 et 90 °C, préférentiellement comprise entre 70 et 80 °C, pour conduire, après lavage par un solvant organique approprié, alcalinisation et extraction par un solvant organique approprié à un dérivé de formule (II/2b) :

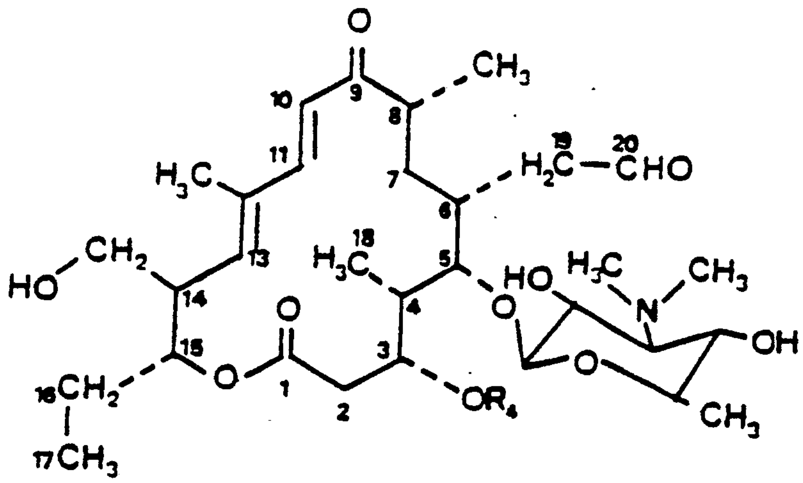

(II/2b)

dans laquelle :

$R_4$ a la même signification que dans la formule (I), que l'on purifie éventuellement par chromatographie sur colonne de silice,

le dérivé alors choisi de formule générale (II), ou (II/2a), ou (II/2b) selon le composé de formule (I) que l'on souhaite obtenir étant alors traité par un cyano phosphonate de dialkyle tel que le cyano phosphonate de diéthyle,

en présence d'une amine de formule (III) HNDE où D et E ont la même signification que dans la formule (I) dans un solvant organique, préférentiellement choisi parmi tétrahydrofuranne, éther diéthylique, éther diisopropylique, benzène, dioxanne, acétone, acétate d'éthyle,

à une température comprise inclusivement entre la température ambiante et la température du reflux du solvant choisi pour conduire à un dérivé de formule (I/A), cas particulier des dérivés de formule (I) pour lesquels G représente un atome d'oxygène, A un groupement NDE et B un groupement cyano,

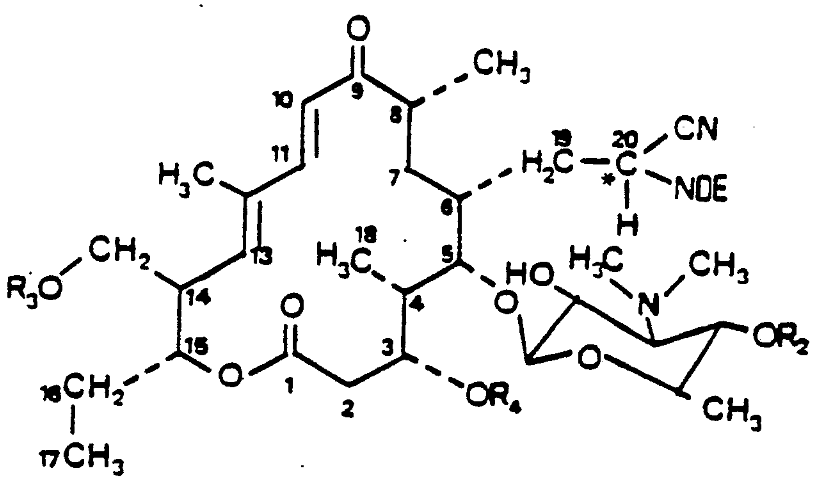

(I/A)

dans laquelle :

D, E et $R_4$ ont la même définition que dans la formule (I), et $R_2$ et $R_3$ la même signification que dans le dérivé choisi (II), (II/1a) ou (II/2b) dont on sépare, si on le désire, les isomères (R) et (S) par une technique classique telle que la chromatographie sur colonne de silice ou la cristallisation fractionnée,

dérivé de formule (I/A) que l'on traite si on le désire en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de oalcium avec un dérivé de formule (V) :

**$H_2N$ - O - $R_1$ (V)**

dans lequel $R_1$ a la même signification que dans la formule (I) ou bien préférentiellement avec un sel d'acide fort (chlorhydrate, bromhydrate...) d'un tel produit pour obtenir après purification éventuelle par chromatographie sur colonne de silice un dérivé de formule (I/D1) :

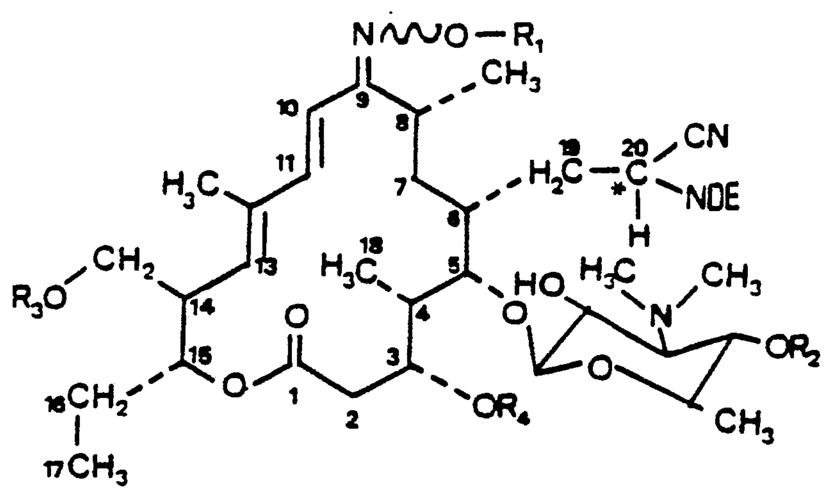

(I/D1)

cas particulier des dérivés de formule (I) dans lesquels G représente un groupement N ∿∿ O - $R_1$ et D, E et $R_4$ ont la même signification que dans la formule du dérivé (I/A),
- que l'on purifie éventuellement par chromatographie sur colonne de silice à l'aide d'un mélange solvant approprié comme par exemple un mélange chlorure de méthylène/méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

3/ Procédé de préparation de composés de formule (I), selon la revendication 1, caractérisé en ce que l'on utilise comme matière un dérivé de formule (II) :

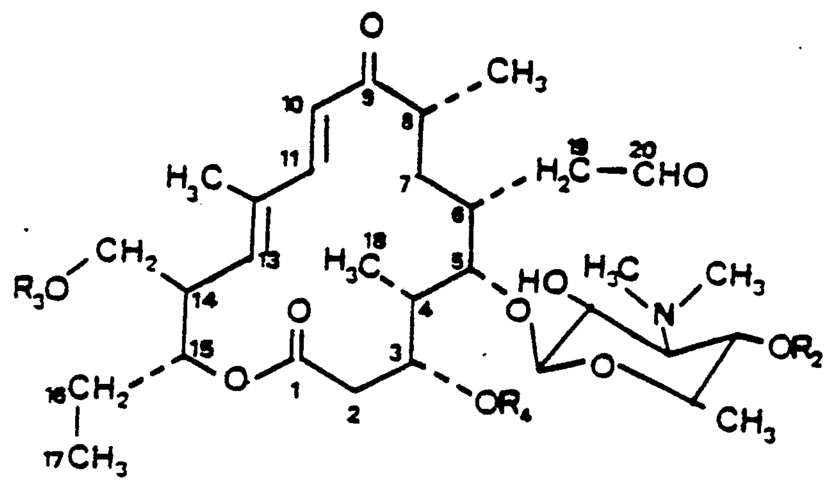

(II)

dans laquelle $R_2$, $R_3$, $R_4$ ont la même définition que dans la formule (I), qui, dans le cas où dans le produit de formule (I) que l'on souhaite obtenir $R_2$ représente un atome d'hydrogène, est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

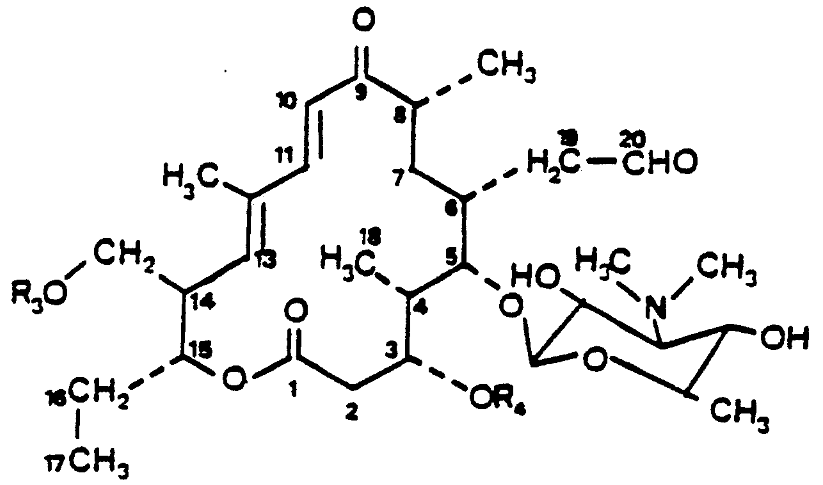

(II/2a)

dans laquelle $R_3$ et $R_4$ ont la même signification que dans la formule (I), qui, dans le cas où dans le produit de formule (I) que l'on souhaite obtenir $R_2$ et $R_3$ simultanément représentent un atome d'hydrogène, est ensuite soumis à réaction avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100 °C, préférentiellement comprise entre 50 et 90 °C, préférentiellement comprise entre 70 et 80 °C, pour conduire, après lavage par un solvant organique approprié, alcalinisation et extraction par un solvant organique approprié à un dérivé de formule (II/2b) :

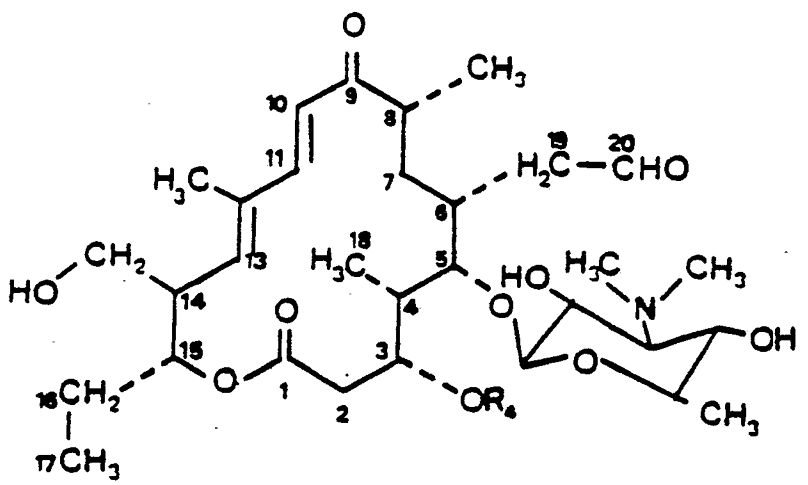

(II/2b)

dans laquelle :

$R_4$ a la même signification que dans la formule (I), que l'on purifie éventuellement par chromatographie sur colonne de silice,

le dérivé alors choisi de formule générale (II), ou (II/2a), ou (II/2b) selon le composé de formule (I) que l'on souhaite obtenir étant alors traité par un dérivé de formule HO - NHD dans lequel D a la même définition que dans la formule (I), ou bien préférentiellement avec un sel d'acide fort d'un tel produit (chlorhydrate, bromhydrate...) dans un solvant organique préférentiellement choisi parmi alcools aliphatiques inférieurs, acétate d'éthyle, acétonitrile en présence d'une base comme par exemple la pyridine, la triéthylamine ou un sel de métal alcalin tel que l'acétate de sodium ou le carbonate acide de sodium ou le carbonate acide de potassium ou le carbonate de sodium, ou le carbonate de potassium ou le carbonate de calcium pour conduire à un dérivé de formule (I/B) :

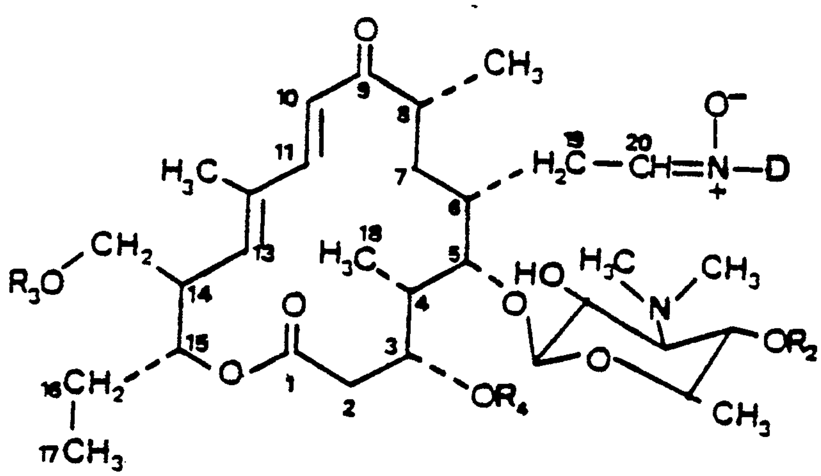

(I/B)

cas particulier des dérivés de formule (I) dans lequel G représente un atome d'oxygène, et A et B représentent simultanément un groupement = $N^+$ -($O^-$) - D,
dans laquelle $R_4$ et D ont la même signification que dans la formule (I) et $R_2$ et $R_3$ la même signification que dans la formule (II), (II/2a) ou (II/2b) du dérivé choisi,
dérivé de formule (I/B) que l'on traite si on le désire en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium avec un dérivé de formule (V) :

**$H_2N$ - O - $R_1$ (V)**

dans lequel $R_1$ a la même signification que dans la formule (I) ou bien préférentiellement avec un sel d'acide fort (chlorhydrate, bromhydrate...) d'un tel produit pour obtenir après purification éventuelle par chromatographie sur colonne de silice un dérivé de formule (I/D2) :

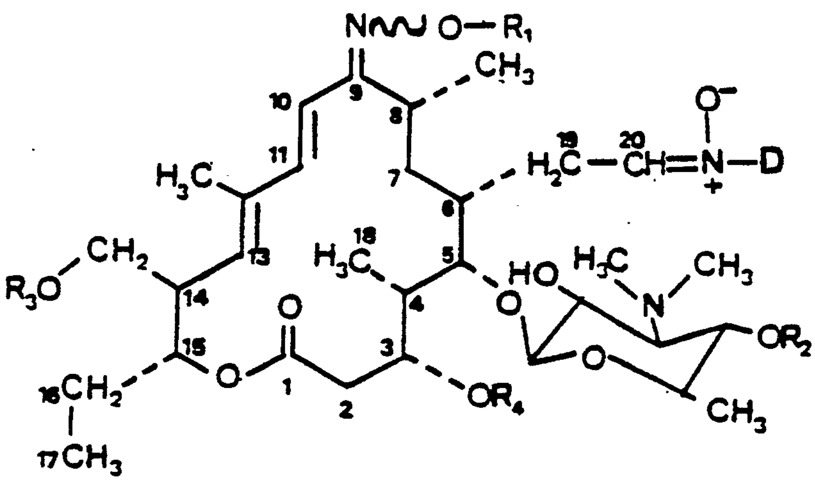

(I/D2)

cas particulier des dérivés de formule (I) dans lesquels G représente un groupement N ∿ O - $R_1$ et A, B et $R_4$ ont la même signification que dans la formule du dérivé (I/B),
- que l'on purifie éventuellement par chromatographie sur colonne de silice à l'aide d'un mélange solvant approprié comme par exemple un mélange chlorure de méthylène/méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

4/ Procédé selon la revendication 1 de préparation de composés de formule (I), dans laquelle A représente un groupement NDE et B représente un groupement cyano, leurs isomères, diastéréoisomères, ainsi que leurs sels d'addition à un acide pharmaceutiquement acceptable.

5/ Procédé selon la revendication 1 de préparation de composés de formule (I), caractérisés en ce que A et B représentent simultanément un groupement = $N^+$ ($O^-$) - D, leurs isomères, diastéréoisomères, ainsi

que leurs sels d'addition à un acide pharmaceutiquement acceptable.

6/ Procédé selon la revendication 1 de préparation de composés de formule (I), caractérisés en ce que G représente un atome d'oxygène, leurs isomères, diastéréoisomères, ainsi que leurs sels d'addition à un acide pharmaceutiquement acceptable.

7/ Procédé selon la revendication 1, de préparation de composés de formule (I), caractérisés en ce que G représente un groupement N ∿ $OR_1$, leurs isomères, diastéréoisomères, ainsi que leurs sels d'addition à un acide pharmaceutiquement acceptable.

8/ Procédé selon la revendication 1, de préparation de composés de formule (I), qui est la benzyloxyimino (E + Z) - 9 dibenzylamino - 20 cyano - 20 didesoxo - 9,20 tylosine.

9/ Procédé selon la revendication 1, de préparation de composés de formule (I), qui est la N benzyl nitrone en 20 de la benzyloxyimino - 9 (E + Z) didesoxo - 9,20 tylosine.

10/ Procédé selon la revendication 1, de préparation de composés de formule (I), qui est la para nitro benzyloxyimino (E + Z) - 9 (R, S) benzylamino - 20 cyano - 20 didesoxo - 9,20 tylosine, ainsi que chacun de ses isomères R et S.

Revendications pour l'Etat contractant suivant: GR

1/ Procédé de préparation de composés de formule (I) :

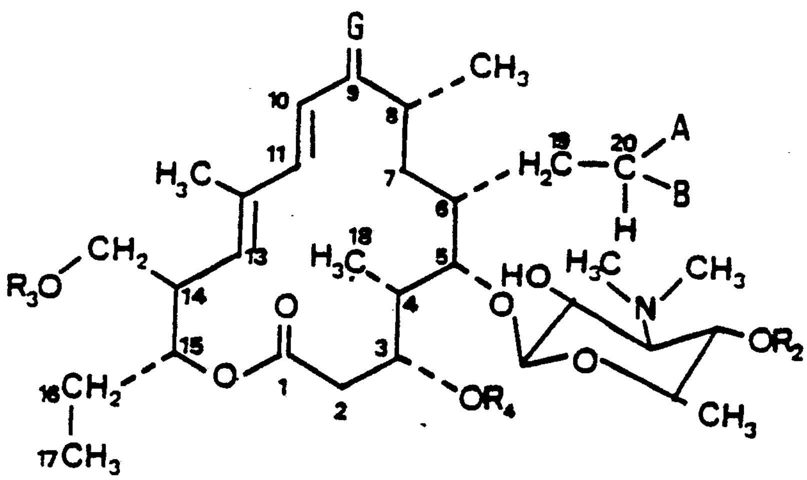

(I)

dans laquelle :

ou bien :

A représente un groupement

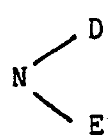

dans lequel D et E identiques ou différents représentent indépendamment l'un de l'autre un atome d'hydrogène, un radical alcoyle inférieur linéaire ou ramifié, éventuellement substitué par un groupement phényle et dans ce cas B représente un groupement cyano,

ou bien :

A et B représentent ensemble un groupement = $N^+$ $(O^-)$ - D dans lequel D a la même signification que précédemment,

G représente un atome d'oxygène ou un groupement de formule N ∿ O -$R_1$ dans lequel $R_1$ représente un atome d'hydrogène, un groupement alcoyle inférieur linéaire ou ramifié, un groupement alkényle inférieur linéaire ou ramifié, ceux-ci étant optionnellement substitué par un groupement phényle lui-même éventuellement substitué par un groupement nitro,

le signe ∿ existant dans la définition de G signifiant que la fonction oxime ou éther d'oxime peut se trouver sous la forme syn ou anti ou sous forme du mélange syn et anti,

$R_2$ représente . soit un atome d'hydrogène,

. soit un radical de formule :

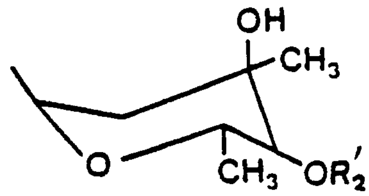

dans lequel $R'_2$ représente un atome d'hydrogène, un radical alcoyle inférieur linéaire ou ramifié ou un radical acyle inférieur, linéaire ou ramifié,
$R_3$ représente un atome d'hydrogène ou un groupement :

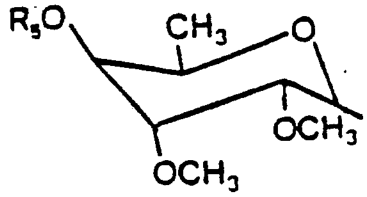

$R_4$ et $R_5$ représentent indépendamment l'un de l'autre :
. soit un atome d'hydrogène,
. soit un radical alcoyle inférieur linéaire ou ramifié,
étant entendu que par radicaux alcoyle inférieur et alkényle inférieur, on entend des groupements comprenant entre 1 et 6 atomes de carbone,
leurs isomères, diastéréoisomères ainsi que leurs sels d'addition à un acide pharmaceutiquement acceptable,
procédé caractérisé en ce que l'on utilise comme matière première un dérivé de formule (II) :

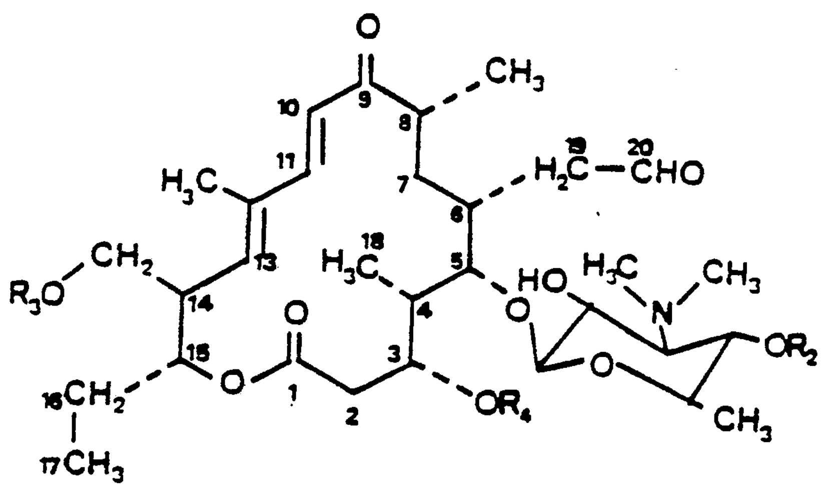

(II)

dans laquelle $R_2$, $R_3$, $R_4$ ont la même définition que dans la formule (I),
qui, dans le cas où dans le produit de formule (I) que l'on souhaite obtenir $R_2$ représente un atome d'hydrogène, est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

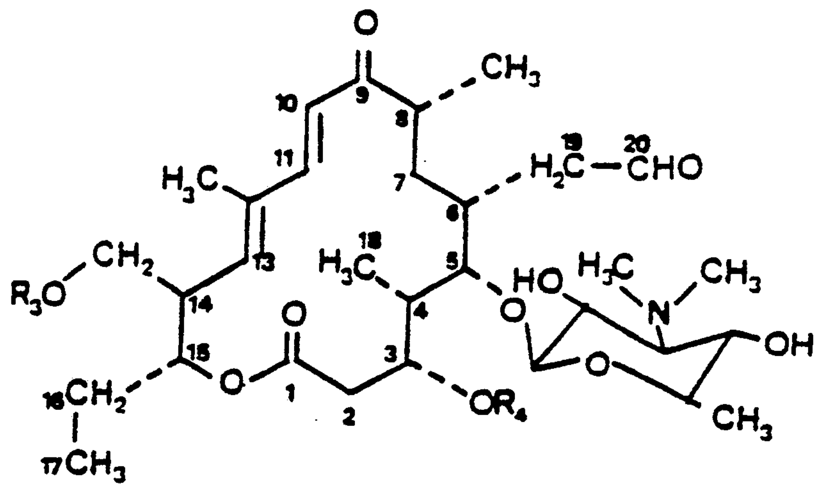

(II/2a)

dans laquelle $R_3$ et $R_4$ ont la même signification que dans la formule (I), qui, dans le cas où dans le produit de formule (I) que l'on souhaite obtenir $R_2$ et $R_3$ simultanément représentent un atome d'hydrogène, est ensuite soumis à réaction avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100 °C, préférentiellement comprise entre 50 et 90 °C, préférentiellement comprise entre 70 et 80 °C, pour conduire, après lavage par un solvant organique approprié, alcalinisation et extraction par un solvant organique approprié à un dérivé de formule (II/2b) :

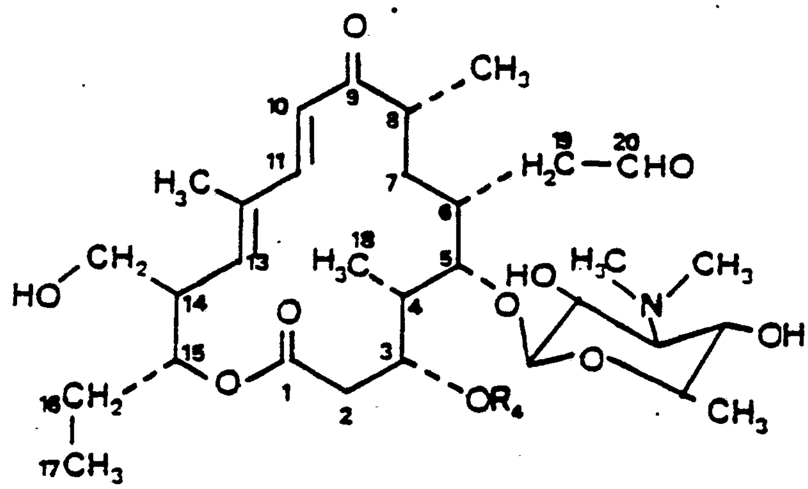

(II/2b)

dans laquelle :
$R_4$ a la même signification que dans la formule (I), que l'on purifie éventuellement par chromatographie sur colonne de silice,
le dérivé alors choisi de formule générale (II), ou (II/2a), ou (II/2b) selon le composé de formule (I) que l'on souhaite obtenir étant alors traité :
- soit, dans le cas où B représente un groupement cyano, par un cyano phosphonate de dialkyle tel que le cyano phosphonate de diéthyle,
en présence d'une amine de formule (III) HNDE où D et E ont la même signification que dans la formule (I) dans un solvant organique, préférentiellement choisi parmi tétrahydrofuranne, éther diéthylique, éther diisopropylique, benzène, dioxanne, acétone, acétate d'éthyle,
à une température comprise inclusivement entre la température ambiante et la température du reflux du solvant choisi pour conduire à un dérivé de formule (I/A), cas particulier des dérivés de formule (I) pour lesquels G représente un atome d'oxygène, A un groupement NDE et B un groupement cyano,

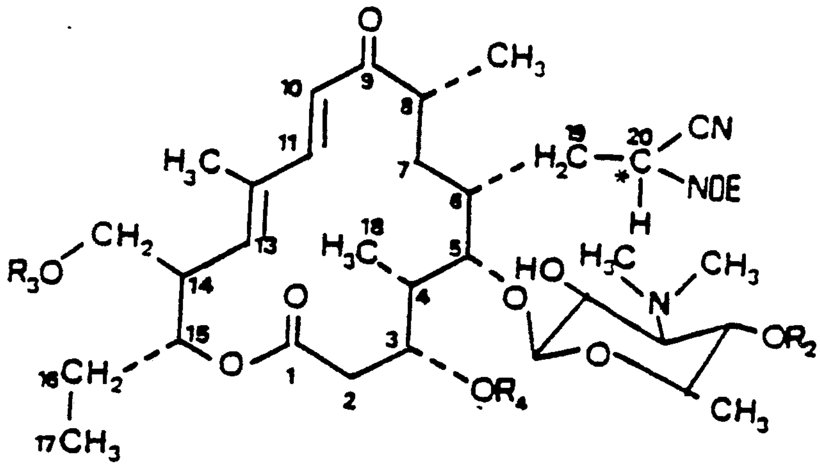

(I/A)

dans laquelle :
D, E et $R_4$ ont la même définition que dans la formule (I), et $R_2$ et $R_3$ la même signification que dans le dérivé choisi (II), (II/2a) ou (II/2b) dont on sépare, si on le désire, les isomères (R) et (S) par une technique classique telle que la chromatographie sur colonne de silice ou la cristallisation fractionnée,
- soit dans le cas où A et B simultanément représentent un groupement = $\overset{+}{N}$ ($O^-$) - D par un dérivé de formule HO - NHD dans lequel D a la même définition que dans la formule (I), ou bien préférentiellement avec un sel d'acide fort d'un tel produit (chlorhydrate, bromhydrate...) dans un solvant organique préférentiellement choisi parmi alcools aliphatiques inférieurs, acétate d'éthyle, acétonitrile en présence d'une base comme par exemple la pyridine, la triéthylamine ou un sel de métal alcalin tel que l'acétate de sodium ou le carbonate acide de sodium ou le carbonate acide de potassium ou le carbonate de sodium, ou le carbonate de potassium ou le carbonate de calcium pour conduire à un dérivé de formule (I/B) :

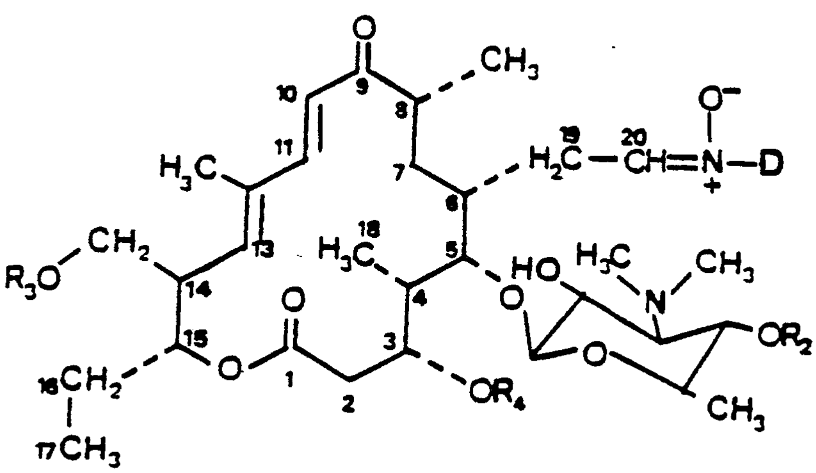

(I/B)

cas particulier des dérivés de formule (I) dans lequel G représente un atome d'oxygène, et A et B représentent simultanément un groupement = $\overset{+}{N}$ -($O^-$) - D,
dans laquelle $R_4$ et D ont la même signification que dans la formule (I) et $R_2$ et $R_3$ dans la même signification que dans la formule (II), (II/2a) ou (II/2b) du dérivé choisi,
dérivé de formule (I/A) ou (I/B) que l'on traite si on le désire en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium avec un dérivé de formule (V) :

**$H_2N$ - O - $R_1$ (V)**

dans lequel $R_1$ a la même signification que dans la formule (I) ou bien préférentiellement avec un sel d'acide fort (chlorhydrate, bromhydrate...) d'un tel produit pour obtenir après purification éventuelle par chromatographie sur colonne de silice un dérivé de formule (I/D) :

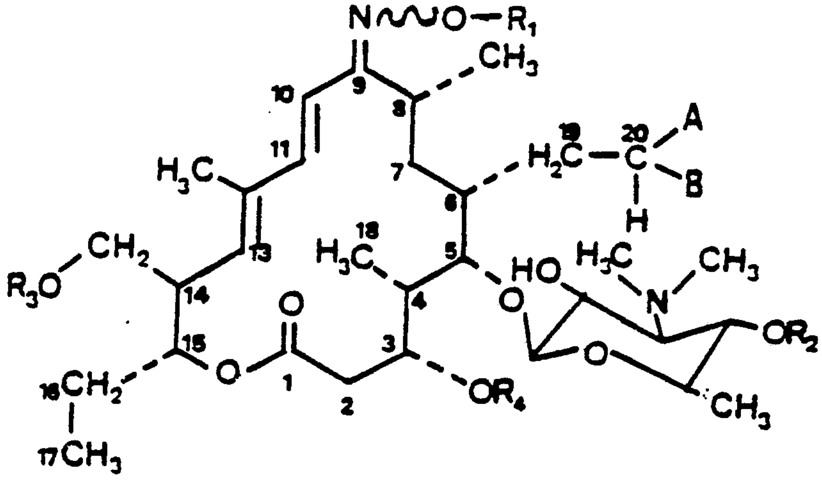

(I/D)

cas particulier des dérivés de formule (I) dans lesquels G représente un groupement N O - $R_1$ et A, B et $R_4$ ont la même signification que dans le dérivé de formules (I/A) ou (I/B),
- que l'on purifie éventuellement par chromatographie sur colonne de silice à l'aide d'un mélange solvant approprié comme par exemple un mélange chlorure de méthylène/méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

2/ Procédé de préparation de composés de formule (I), selon la revendication 1, caractérisé en ce que l'on utilise comme matière première un dérivé de formule (II) :

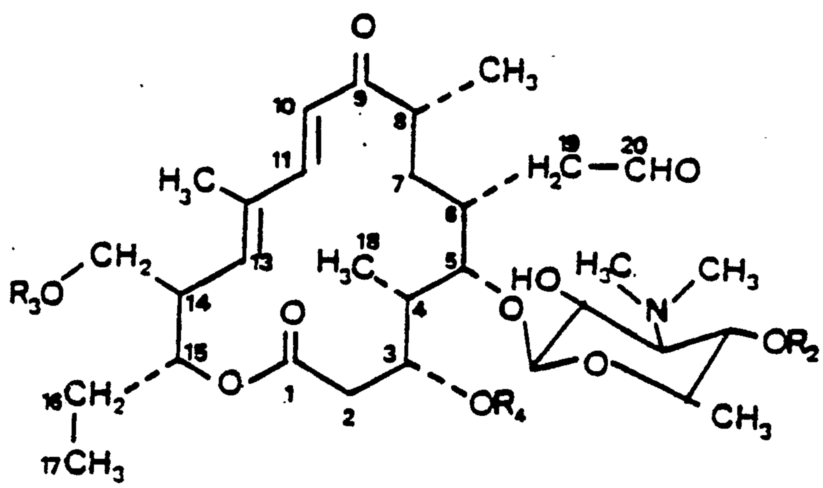

(II)

dans laquelle $R_2$, $R_3$, $R_4$ ont la même définition que dans la formule (I),
qui, dans le cas où dans le produit de formule (I) que l'on souhaite obtenir $R_2$ représente un atome d'hydrogène, est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

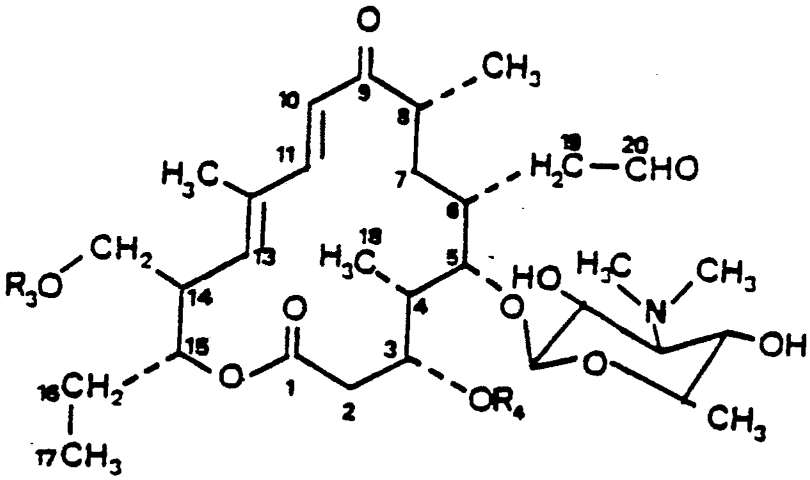

(II/2a)

dans laquelle $R_3$ et $R_4$ ont la même signification que dans la formule (I), qui, dans le cas où dans le produit de formule (I) que l'on souhaite obtenir $R_2$ et $R_3$ simultanément représentent un atome d'hydrogène, est ensuite soumis à réaction avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100 °C, préférentiellement comprise entre 50 et 90 °C, préférentiellement comprise entre 70 et 80 °C, pour conduire, après lavage par un solvant organique approprié, alcalinisation et extraction par un solvant organique approprié à un dérivé de formule (II/2b) :

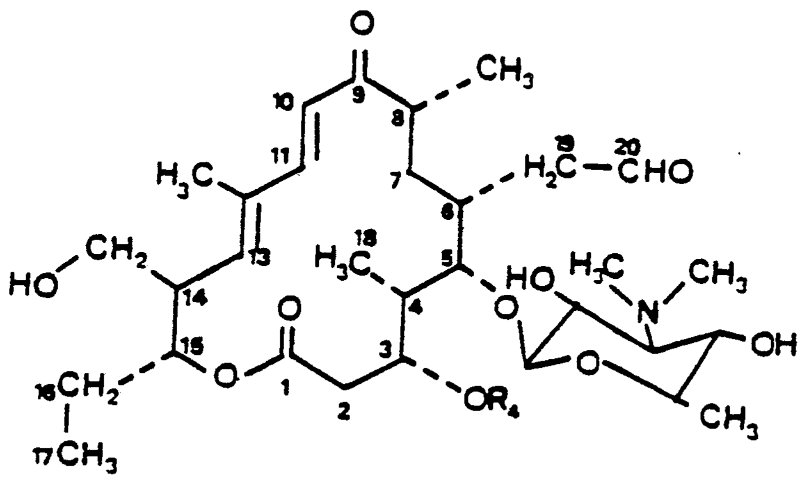

(II/2b)

dans laquelle :
$R_4$ a la même signification que dans la formule (I), que l'on purifie éventuellement par chromatographie sur colonne de silice,
le dérivé alors choisi de formule générale (II), ou (II/2a), ou (II/2b) selon le composé de formule (I) que l'on souhaite obtenir étant alors traité par un cyano phosphonate de dialkyle tel que le cyano phosphonate de diéthyle,
en présence d'une amine de formule (III) HNDE où D et E ont la même signification que dans la formule (I) dans un solvant organique, préférentiellement choisi parmi tétrahydrofuranne, éther diéthylique, éther diisopropylique, benzène, dioxanne, acétone, acétate d'éthyle,
à une température comprise inclusivement entre la température ambiante et la température du reflux du solvant choisi pour conduire à un dérivé de formule (I/A), cas particulier des dérivés de formule (I) pour lesquels G représente un atome d'oxygène, A un groupement NDE et B un groupement cyano,

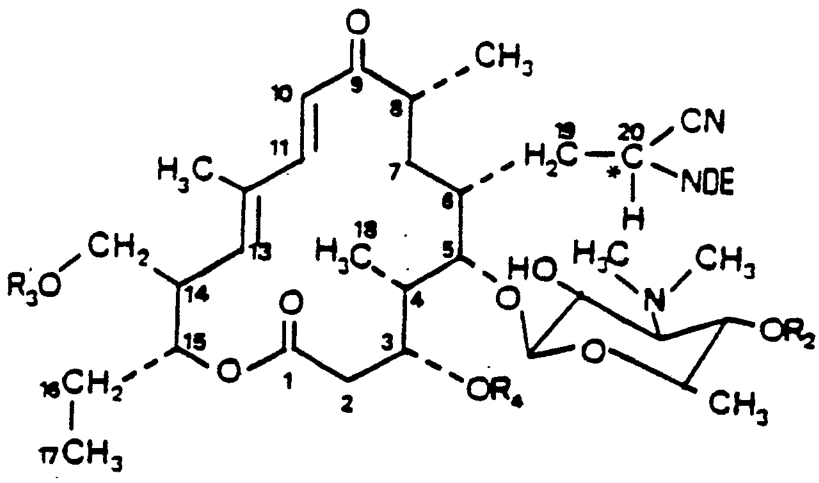

(I/A)

dans laquelle :
D, E et $R_4$ ont la même définition que dans la formule (I), et $R_2$ et $R_3$ la même signification que dans le dérivé choisi (II), (II/1a) ou (II/2b) dont on sépare, si on le désire, les isomères (R) et (S) par une technique classique telle que la chromatographie sur colonne de silice ou la cristallisation fractionnée,
dérivé de formule (I/A) que l'on traite si on le désire en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium avec un dérivé de formule (V) :

**$H_2N$ - O - $R_1$ (V)**

dans lequel $R_1$ a la même signification que dans la formule (I) ou bien préférentiellement avec un sel d'acide fort (chlorhydrate, bromhydrate...) d'un tel produit pour obtenir après purification éventuelle par chromatographie sur colonne de silice un dérivé de formule (I/D1) :

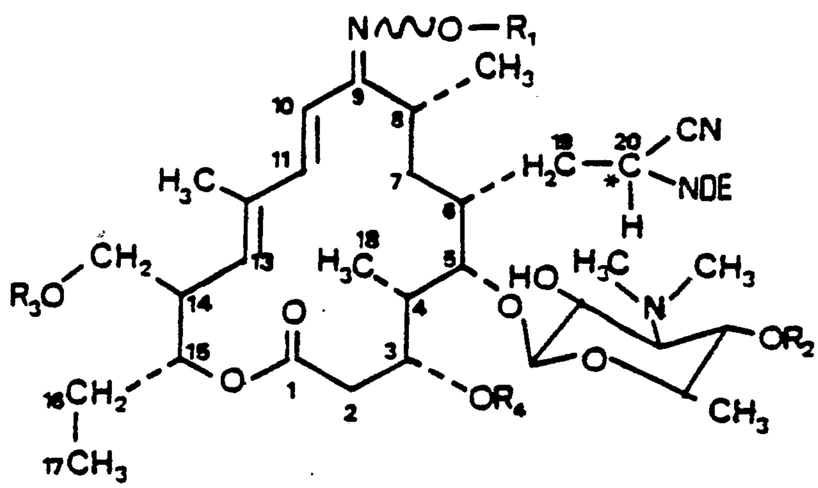

(I/D1)

cas particulier des dérivés de formule (I) dans lesquels G représente un groupement N ~ O - $R_1$ et D, E et $R_4$ ont la même signification que dans la formule du dérivé (I/A),
- que l'on purifie éventuellement par chromatographie sur colonne de silice à l'aide d'un mélange solvant approprié comme par exemple un mélange chlorure de méthylène/méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

3/ Procédé de préparation de composés de formule (I), selon la revendication 1, caractérisé en ce que l'on utilise comme matière un dérivé de formule (II) :

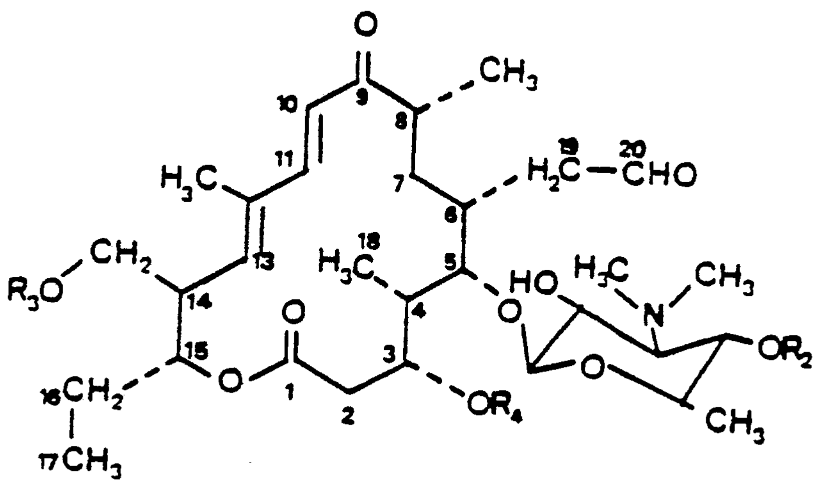

(II)

dans laquelle $R_2$, $R_3$, $R_4$ ont la même définition que dans la formule (I), qui, dans le cas où dans le produit de formule (I) que l'on souhaite obtenir $R_2$ représente un atome d'hydrogène, est tout d'abord soumis à l'action de l'acide chlorhydrique dilué de normalité comprise entre 0,05 et 0,4, préférentiellement comprise entre 0,1 et 0,30, préférentiellement comprise entre 0,15 et 0,25, à température ambiante pour conduire après lavage par un solvant organique approprié, alcalinisation et extraction par un solvant organique approprié à un dérivé de formule (II/2a) :

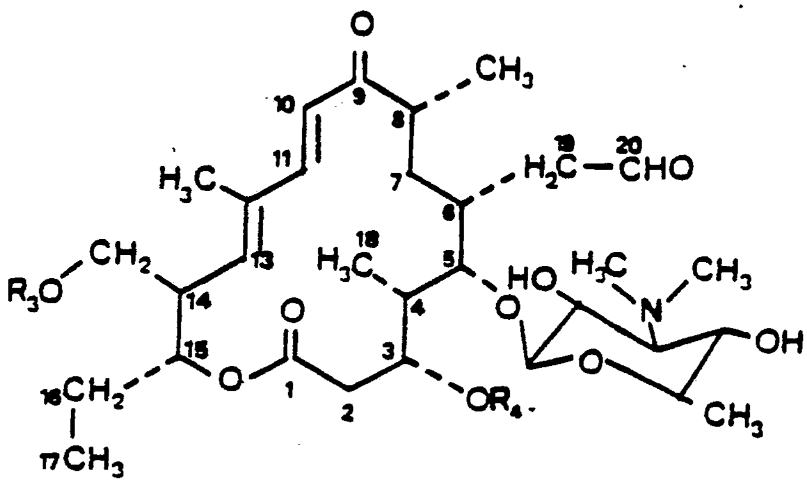

(II/2a)

dans laquelle $R_3$ et $R_4$ ont la même signification que dans la formule (I), qui, dans le cas où dans le produit de formule (I) que l'on souhaite obtenir $R_2$ et $R_3$ simultanément représentent un atome d'hydrogène, est ensuite soumis à réaction avec l'acide chlorhydrique dilué de normalité comprise entre 0,25 et 0,75, préférentiellement comprise entre 0,3 et 0,7, préférentiellement comprise entre 0,4 et 0,6, à une température préférentiellement comprise entre 30 et 100 °C, préférentiellement comprise entre 50 et 90 °C, préférentiellement comprise entre 70 et 80 °C, pour conduire, après lavage par un solvant organique approprié, alcalinisation et extraction par un solvant organique approprié à un dérivé de formule (II/2b) :

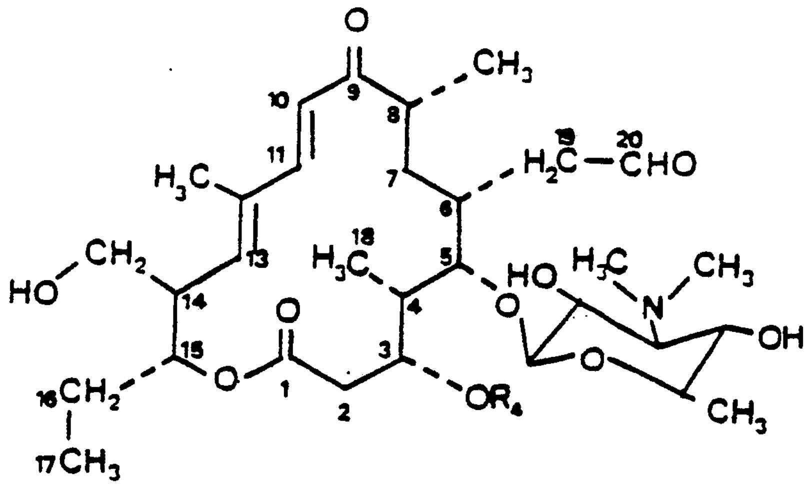

(II/2b)

dans laquelle :

$R_4$ a la même signification que dans la formule (I), que l'on purifie éventuellement par chromatographie sur colonne de silice,

le dérivé alors choisi de formule générale (II), ou (II/2a), ou (II/2b) selon le composé de formule (I) que l'on souhaite obtenir étant alors traité par un dérivé de formule HO - NHD dans lequel D a la même définition que dans la formule (I), ou bien préférentiellement avec un sel d'acide, fort d'un tel produit (chlorhydrate, bromhydrate...) dans un solvant organique préférentiellement choisi parmi alcools aliphatiques inférieurs, acétate d'éthyle, acétonitrile en présence d'une base comme par exemple la pyridine, la triéthylamine ou un sel de métal alcalin tel que l'acétate de sodium ou le carbonate acide de sodium ou le carbonate acide de potassium ou le carbonate de sodium, ou le carbonate de potassium ou le carbonate de calcium pour conduire à un dérivé de formule (I/B) :

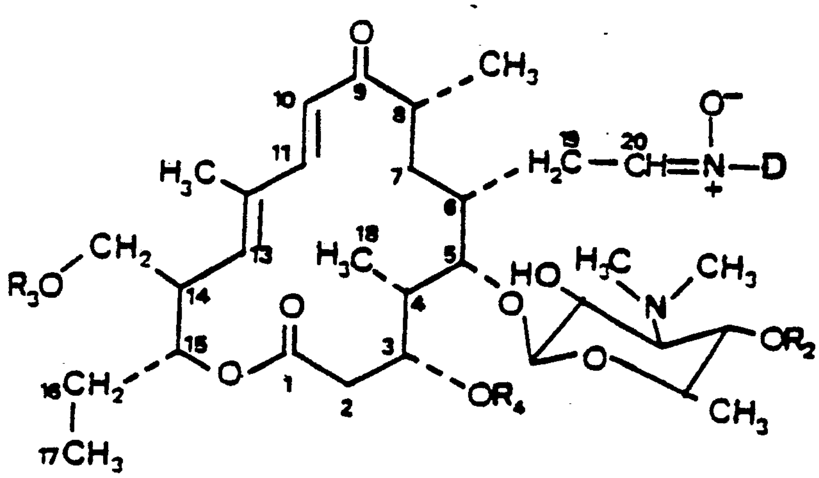

(I/B)

cas particulier des dérivés de formule (I) dans lequel G représente un atome d'oxygène, et A et B représentent simultanément un groupement $= N^{+} -(O^{-}) - D$,

dans laquelle $R_4$ et D ont la même signification que dans la formule (I) et $R_2$ et $R_3$ la même signification que dans la formule (II), (II/2a) ou (II/2b) du dérivé choisi,

dérivé de formule (I/B) que l'on traite si on le désire en présence d'une base, comme par exemple la pyridine, la triéthylamine ou un sel de métal alcalin tel que l'acétate de sodium ou le carbonate acide de sodium ou le carbonate acide de potassium, ou le carbonate de sodium, ou le carbonate de potassium ou le carbonate de calcium avec un dérivé de formule (V) :

**$H_2N - O - R_1$ (V)**

dans lequel $R_1$ a la même signification que dans la formule (I) ou bien préférentiellement avec un sel d'acide fort (chlorhydrate, bromhydrate...) d'un tel produit pour obtenir après purification éventuelle par chromatographie sur colonne de silice un dérivé de formule (I/D2) :

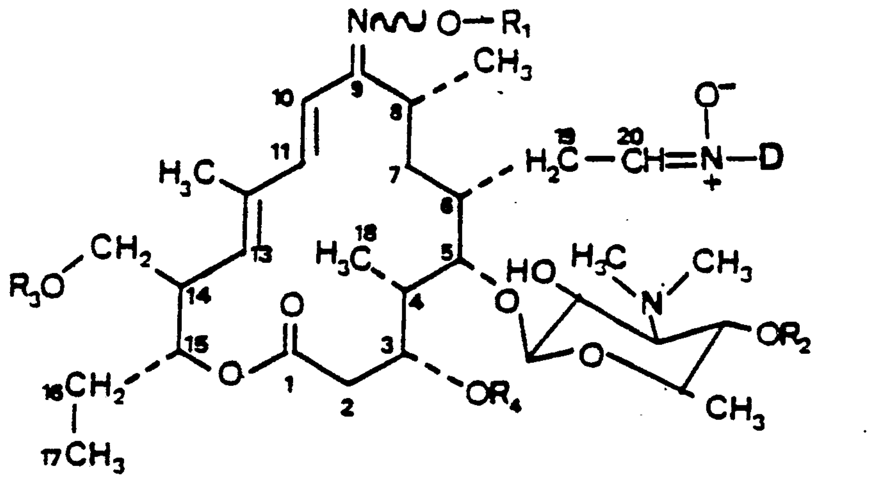

(I/D2)

cas particulier des dérivés de formule (I) dans lesquels G représente un groupement N ∿∿ O - $R_1$ et A, B et $R_4$ ont la même signification que dans la formule du dérivé (I/B),
- que l'on purifie éventuellement par chromatographie sur colonne de silice à l'aide d'un mélange solvant approprié comme par exemple un mélange chlorure de méthylène/méthanol,
et que l'on peut, si on le désire :
- soit salifier par un acide pharmaceutiquement acceptable,
- soit séparer en ses isomères puis, si nécessaire, salifier par un acide pharmaceutiquement acceptable.

4/ Procédé selon la revendication 1 de préparation de composés de formule (I), dans lequelle A représente un groupement NDE et B représente un groupement cyano, leurs isomères, diastéréoisomères, ainsi que leurs sels d'addition à un acide pharmaceutiquement acceptable.

5/ Procédé selon la revendication 1 de préparation de composés de formule (I), caractérisés en ce que A et B représentent simultanément un groupement = $N^+$ ($O^-$) - D, leurs isomères, diastéréoisomères, ainsi que leurs sels d'addition à un acide pharmaceutiquement acceptable.

6/ Procédé selon la revendication 1 de préparation de composés de formule (I), caractérisés en ce que G représente un atome d'oxygène, leurs isomères, diastéréoisomères, ainsi que leurs sels d'addition à un acide pharmaceutiquement acceptable.

7/ Procédé selon la revendication 1, de préparation de composés de formule (I), caractérisés en ce que G représente un groupement N ∿∿ $OR_1$, leurs isomères, diastéréoisomères, ainsi que leurs sels d'addition à un acide pharmaceutiquement acceptable.

8/ Procédé selon la revendication 1, de préparation de composés de formule (I), qui est la benzyloxyimino (E + Z) - 9 dibenzylamino - 20 cyano - 20 didesoxo - 9,20 tylosine.

9/ Procédé selon la revendication 1, de préparation de composés de formule (I), qui est la N benzyl nitrone en 20 de la benzyloxyimino - 9 (E + Z) didesoxo - 9,20 tylosine.

10/ Procédé selon la revendication 1, de préparation de composés de formule (I), qui est la para nitro benzyloxyimino (E + Z) - 9 (R, S) benzylamino - 20 cyano - 20 didesoxo - 9,20 tylosine, ainsi qui chacun de ses isomères R et S.

Office européen des brevets

# RAPPORT DE RECHERCHE EUROPEENNE

Numero de la demande

EP 89 40 2969

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 287 082 (SOUR PLIVA)<br>* Pages 16-17 *<br>--- | 1,3-5,9,11-13 | C 07 H 17/08<br>A 61 K 31/70 |
| X | CHEMICAL ABSTRACTS, vol. 107, 1987, page 697, résumé no. 7519n, Columbus, Ohio, US; & JP-A-61 191 692 (TOYO JOZO CO., LTD) 26-08-1986; * Résumé * & CHEMICAL ABSTRACTS, CHEMICAL SUBSTANCE INDEX, Q-Z, vol. 107, juillet-décembre 1987, pages 9491CS, 9492CS; Tylosin, -, 4A-O-acetyl-4A-O-de(2,6-dideoxy-3-C-methyl-alpha-L-ribo-hexopyranosyl)-20-oxime, 2A-acetate [108521-04-6], prepn. and cyanation of, P. 7519n. 20-oxime, 2A,4C-diacetate [108491-90-3], prepn. and selective deacetylation of, P 7519n. -, 4A-O-de(2,6-dideoxy-3-C-methyl-alpha-L-ribo-hexopyranosyl)-(desmycosin, tylosin B) [11032-98-7] formation of by streptomyces fradiae, fatty cid compn. in relation to, 150860x prepn. of 9-O-acyl derivs. of 237175b. Wittig methylenation of, P 7519n. 20-oxime, (20E)- [108590-21-2], prepn. and acetylation of, P 7519n. 20-oxime, (20Z)- [108491-89-0], prepn. and acetylation of, P 7519n.<br>--- | 1-5,9,11-13 | |
| Y | CHEMICAL ABSTRACTS, vol. 108, 1988, page 754, résumé no. 187201e, Columbus, Ohio, US; & JP-A-62 221 695 (NATIONAL INSTITUTE FOR RESEARCH IN INORGANIC MATERIALS) 29-09-1987<br>* Résumé *<br>--- -/- | 1,3,11 | |

| | | | DOMAINES TECHNIQUES RECHERCHES (Int. Cl.5) |
|---|---|---|---|
| | | | C 07 H 17/00<br>A 61 K 31/00 |

Le présent rapport a été établi pour toutes les revendications

| Lieu de la recherche | Date d'achèvement de la recherche | Examinateur |
|---|---|---|
| LA HAYE | 18-12-1989 | VERHULST W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 89 40 2969

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | CHEMICAL ABSTRACTS, vol. 90, 1979, page 550, résumé no. 138149v, Columbus, Ohio, US; & JP-A-78 132 584 (TOYO JOZO CO., LTD) 18-11-1978<br>* Résumé *<br>--- | 1,2,11 | |
| Y | EP-A-0 087 921 (S. OMURA)<br>* Pages 24-27 *<br>----- | 1,2,11 | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| LA HAYE | 18-12-1989 | VERHULST W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant